(19) Europäisches Patentamt
European Patent Office
Office européen des brevets

(11) Publication number: **0 325 406 B1**

(12) **EUROPEAN PATENT SPECIFICATION**

(45) Date of publication of patent specification: **29.12.93**

(51) Int. Cl.5: **C07C 233/01**, C07D 295/14, C07D 295/12, A61K 31/40

(21) Application number: **89300400.2**

(22) Date of filing: **17.01.89**

(54) **Diamine compounds.**

(30) Priority: **21.01.88 GB 8801304**

(43) Date of publication of application:
**26.07.89 Bulletin 89/30**

(45) Publication of the grant of the patent:
**29.12.93 Bulletin 93/52**

(84) Designated Contracting States:
**BE CH DE ES LI NL**

(56) References cited:
**EP-A- 0 110 869**
**EP-A- 0 126 612**
**EP-A- 0 254 545**
**EP-A- 0 261 842**
**FR-A- 2 370 723**

**CHEMICAL ABSTRACTS, vol. 109, no. 17, 24th October 1988, page 53, abstract no. 142412d, Columbus, Ohio, US; G.F. COSTELLO et al.: "A novel series of potent and selective agonists at the opioid kappa-receptor"**

**CHEMICAL ABSTRACTS, vol. 111, no. 3, 17th July 1989, page 47, abstract no. 17521d, Columbus, Ohio, US; J.S. SHAW et al.: "ICI 204448: a kappa-opioid agonist with limited access to the CNS"**

(73) Proprietor: **IMPERIAL CHEMICAL INDUSTRIES PLC**
**Imperial Chemical House,**
**Millbank**
**London SW1P 3JF(GB)**

(72) Inventor: **Main, Brian Geoffrey**
**26 Coldmoss Drive**
**Sandbach**
**Cheshire(GB)**

(74) Representative: **Mack, John Richard et al**
**ICI Group Patents Services Dept.**
**PO Box 6**
**Shire Park**
**Bessemer Road**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 325 406 B1

Rank Xerox (UK) Business Services
(3.10/3.6/3.3.1)

## Description

The present invention relates to 1,2-ethylene diamine compounds and the salts thereof, processes for their preparation and pharmaceutical compositions containing the said compounds and the pharmaceutically acceptable salts thereof. The compounds and their pharmaceutically acceptable salts possess a selective effect on peripheral kappa opiate receptors.

US Patent specification No. 4,145,435 discloses cis- and trans-N-(2-aminocycloaliphatic)-2-arylacetamide derivatives as possessing potent analgesic activity. European Patent Publication No. 110 869 discloses trans-N-(2-aminocyclohexyl)-2-thienylacetamide derivatives and European Patent Publication No. 126,612 discloses cis-and trans-N-[2-(2,5-dihydro-1H-pyrrol-1- yl)cycloaliphatic]-2-benzeneacetamide derivatives all possessing analgesic activity.

Our copending European Patent Application No 254545 describes and claims certain open chain ethylene diamine compounds which possess a significantly improved analgesic potency over corresponding known compounds. The invention in our aforementioned copending European Patent Application is based on the discovery that the cycloaliphatic ring present in the above-mentioned compounds is not essential for activity, and further on the discovery that provided the stereochemical centre is maintained at the carbon adjacent to the amidic nitrogen atom, the open chain ethylene diamine structure enables a wide range of substituents to be introduced which would not otherwise have been possible, whereby compounds having a significantly improved analgesic potency over corresponding known compounds may be obtained.

The present invention is based on the discovery that compounds possessing a selective effect on peripheral kappa receptors are obtained if a phenyl group substituted in the meta-position by a radical comprising an organic base, an organic acid or a quaternary ammonium salt (and optionally further substituted), is present on the carbon adjacent to the amidic nitrogen atom of the aforementioned open chain ethylene diamine compounds, the meta-substituent being selected such that the compounds of the invention have a log D of less than 0.5 at pH 7.4 (where log D is the logarithm of the distribution coefficient between octan-1-ol and aqueous buffer). The side-effects associated with centrally acting kappa agonists such as dysphoria and other CNS problems are thus significantly ameliorated in the compounds of the present invention.

According to one feature of the present invention there are provided compounds of formula I (as set out hereinafter) [wherein $R^1$ represents a halophenyl, dihalophenyl, nitrophenyl, cyanophenyl or trifluoromethylphenyl group:

X represents a single bond,$-CH_2-$, $OCH_2-$, $-SCH_2-$, $-S(O)-CH_2-$, $-S(O)_2-CH_2-$ or $-CH_2-CH_2-$;

$R^2$ represents hydrogen or $C_{1-3}$ alkyl;

$R^3$ represents a phenyl group substituted in the meta position by a radical containing an organic base, an organic acid or a quaternary ammonium salt and optionally further substituted by halogen, $C_{1-3}$alkyl, $C_{1-3}$alkoxy, cyano, nitro, hydroxy, amino, mono- or di-($C_1$ or $_2$alkyl)amino, carboxamido, mono- or di-($C_1$ or $_2$alkyl)carboxamido, ureido or $C_{1-3}$acylamino;

$R^4$ and $R^5$, which may be the same or different, each represents a $C_{3-5}$ alkenyl, $C_{3-5}$ alkynyl, $C_{1-6}$ alkyl, or $C_{4-7}$ cycloalkylalkyl group;

or $R^4$ and $R^5$ together with the intervening nitrogen atom represent a 4-7-membered heterocyclic ring which optionally contains a further heteroatom selected from oxygen and sulphur] or racemates thereof, and the salts of said compounds or racemates; the group $-R^3$ being selected such that the compound of formula I or racemate thereof or salt of said compound of formula I or racemate thereof has a log D of less than 0.5 at pH 7.4 (where log D is the logarithm of the distribution coefficient between octan-1-ol and aqueous buffer).

Unless otherwise specified, references herein to alkyl, alkenyl and alkynyl groups include such groups in both straight chain and branched forms.

The compounds of formula I possess an asymmetric carbon atom, that being the carbon atom carrying the $R^3$ substituent in formula I. It will be understood that the present invention includes the racemates of the compounds of formula I as well as the optically active enantiomer having the absolute configuration at the above-mentioned asymmetric carbon atom which would be obtained by synthesis from a corresponding natural (L)-alpha-amino acid as indicated in formula I which compounds possess the useful physiological properties of the compounds of the invention herein described.

Furthermore the substituent $R^3$ may contain at least one asymmetric carbon atom and it will be understood that the present invention encompasses the racemic form of such compounds as well as any individual optical isomers thereof which possess the useful physiological properties of the compounds of the present invention as herein defined, it being common general knowledge to those skilled in the art how such isomers may be separated and how their physiological properties may be determined.

The present invention encompasses the salts of the compounds of formula I or racemate thereof. It will be appreciated, however, that for pharmaceutical use, the salts referred to will be pharmaceutically acceptable, but other salts may find use, for example in the preparation of compounds of formula I and their pharmaceutically acceptable salts. The salts of the present invention include acid addition salts with for example mineral acids such as hydrochloric acid and organic acids such as maleic and fumaric acid.

The compounds of the present invention, have a log D of less than 0.5 at pH 7.4. The distribution coefficient (D) between octan-1-ol and aqueous buffer at pH 7.4 of a compound of the present invention may be determined as described hereinafter.

Where $R^4$ and $R^5$ together with the intervening nitrogen atom represents a 4-7 membered ring optionally containing a further heteroatom, particular values for said ring are the pyrrolidinyl, pyrrolinyl, morpholinyl or piperidinyl group.

Particular values for $R^1$ are chlorophenyl such as 3-chlorophenyl or 4-chlorophenyl; dichlorophenyl such as 3,4-dichlorophenyl; bromophenyl such as 4-bromophenyl; or fluorophenyl such as 3-fluorophenyl or 4-fluorophenyl; difluorophenyl such as 3,4-difluorophenyl; trifluoromethylphenyl such as 4-(trifluoromethyl)-phenyl; nitrophenyl such as 4-nitrophenyl; and cyanophenyl such as 4-cyanophenyl.

Particular values for X are $-CH_2-$, $-OCH_2$, $-SCH_2-$ or $-CH_2-CH_2-$, especially $-CH_2-$.

Particular values for $R^2$ are hydrogen, methyl, ethyl or isopropyl, especially methyl.

Particular values for $R^3$ are phenyl substituted in the meta-position by:-

(i) a group of the formula;-

-Y-W-M II

in which Y represents a single bond, -S-, -0- or -NR- (in which R represents hydrogen, methyl, formyl or acetyl), W represents a $C_{2-4}$alkylene chain which may if desired be branched and M represents the group $-NR^7R^8R^9$ (wherein $R^7$, $R^8$ and $R^9$, which may be the same or different, each represents a $C_{1-7}$alkyl or aralkyl group;

(ii) a group of the formula III in which $Y^1$ represents -0- or -S-; W represents a $C_{2-4}$alkylene chain which may if desired be branched; and $R^{10}$, $R^{11}$ and $R^{12}$, which may be the same or different, each represents hydrogen or methyl or $R^{10}$ and $R^{11}$ together represent a $C_2$ or $C_3$ alkylene chain such that $R^{10}$ and $R^{11}$ together with the intervening radical of formula IV form a 4,5-dihydroimidazol-2-yl or 3,4,5,6-tetrahydro-pyrimidin-2-yl ring, $R^{12}$ representing hydrogen or methyl; or $R^{11}$ and $R^{12}$ together represent a $C_4$ or $_5$ alkylene chain such that $R^{11}$ and $R^{12}$ together with the intervening nitrogen atom form a pyrrolidine or piperidine ring and $R^{10}$ represents hydrogen or methyl;

(iii) a group of the formula V in which $W^1$ represents a $C_{2-4}$alkylene chain which may if desired be branched and $R^{10}$, $R^{11}$ and $R^{12}$ are as hereinbefore defined.

(iv) a group of the formula VI in which $W^2$ represents a $C_{1-4}$ alkylene chain which may if desired be branched and $R^{10}$, $R^{11}$ and $R^{12}$ are as hereinbefore defined;

v) a group of the formula VII in which Y is as hereinbefore defined, $W^3$ represents a $C_{2-4}$alkylene chain which may if desired be branched or where Y represents a single bond $W^3$ may represent a single bond or a $C_{1-4}$alkylene chain which may if desired be branched and $R^{10}$, $R^{11}$, $R^{12}$ and $R^{13}$, which may be the same or different, each represents hydrogen or methyl,

or $R^{10}$ and $R^{12}$ together represent a $C_2$ or $_3$ alkylene chain such that $R^{10}$ and $R^{12}$ together with the intervening radical of formula IVa form a 4,5-dihydroimidazol-2-yl or 3,4,5,6-tetrahydropyrimidin-2-yl ring or tautomer thereof, $R^{11}$ and $R^{12}$ being independently selected from hydrogen or methyl,

or either $R^{10}$ and $R^{11}$ together or $R^{12}$ and $R^{13}$ together represent a $C_4$ or $_5$ alkylene chain such that either $R^{10}$ and $R^{11}$ together with the intervening nitrogen atom form a pyrrolidine or piperidine ring and $R^{12}$ and $R^{13}$ are independently selected from hydrogen and methyl or $R^{12}$ and $R^{13}$ together with the intervening nitrogen atom form a pyrrolidine or piperidine ring and $R^{10}$ and $R^{11}$ are independently selected from hydrogen and methyl; or (vi) a group of the formula VIII in which Y and $W^2$ are as hereinbefore defined and A represents a carboxylic or sulphonic acid.

Where $R^3$ represents a phenyl group carrying further substituents in addition to the substituent at the meta-position, such further substituents are particularly selected from chlorine, fluorine, methyl, methoxy, cyano, nitro, hydroxy, amino, monomethylamino, dimethylamino, carboxamido, monomethylcarboxamido, dimethylcarboxamido, ureido and acetylamino. However $R^3$ especially represents a phenyl group which is unsubstituted apart from carrying a substituent at the meta- position as herein defined.

$R^3$ is preferably selected such that the organic base, the organic acid or the quaternary ammonium salt is linked to phenyl at the meta position via oxygen or -NH-.

Particular values for $R^4$ and $R^5$, which may be the same or different, are each a $C_{1-6}$ alkyl group or a $C_{3-5}$ alkenyl group. Particular values for $R^4$ and $R^5$ together with the intervening nitrogen atom are 5- or 6-membered heterocyclic rings, especially where the nitrogen atom is the sole heteroatom.

More particular values for $R^1$ are 3,4-dichlorophenyl and 4-trifluoromethylphenyl, especially 3,4-dichlorophenyl.

A more particular value for $R^2$ is methyl.

More particular values for $R^3$ are phenyl substituted in the meta-position by:

i) a group of formula II in which W is $C_2$ or $C_3$ alkylene;

ii) a group of formula III in which W is $CH_2CH_2$;

iii) a group of formula V in which $W^1$ is a single bond or $-CH_2CH_2-$;

iv) a group of formula VI in which $W^2$ is $-CH_2-$ or $-CH_2CH_2-$;

v) a group of formula VII in which Y represents -0-, -S- or -NR-wherein R is as hereinbefore defined) and $W^3$ is $-CH_2CH_2-$; or a group of formula VII in which Y represents a single bond and $W^3$ is a single bond $-CH_2-$ or $CH_2CH_2-$;

vi) A group of formula VIII in which $W^2$ is $-CH_2-$;

Where the grouping >N-R is present, as in formulae II, VII or VIII in which Y may represent >N-R, or in formula V, R is particularly hydrogen.

Where the groupings $R^7$, $R^8$ or $R^9$ are present, such groupings, which may be the same or different, are particularly selected from methyl and benzyl.

Thus $R^3$ may especially represent 3-(3-dimethylamino-propoxy)phenyl in the form of a quaternary ammonium salt and 3- (3-carboxymethoxy)phenyl and 3-guanidinophenyl. Thus $R^3$ may especially represent the 3-trimethylammoniumpropoxy and 3-benzyldimethylammoniumpropoxy groups stabilised by an appropriate anion such as iodide or chloride.

More particular values for $R^4$ and $R^5$ together with the intervening nitrogen atom are pyrrolidino, dimethylamino, diethylamino, N-allyl-N-methylamino, N-isopropyl-N-methylamino, $\Delta^3$-pyrrolino or piperidino, especially pyrrolidino. Preferred compounds of the present invention, by virtue of their selective effect on peripheral kappa receptors include the following:-2(R,S)-N- [2-(N-methyl-3, 4-dichlorophenylacetamido)-2-(3-carboxymethoxyphenyl)-ethyl]pyrrolidine, (R,S)-N-[2-(N-methyl-3,4-dichlorophenylacetamido)-2-(3-trimethylammoniumpropoxyphenyl)-ethyl]-pyrrolidine iodide, (R,S)-N-[2-(N-methyl-3,4-dichlorophenylacetamido)-2-(3-benzyldimethylammoniumpropoxy-phenyl)-ethyl]-pyrrolidine chloride;
(R,S)-N-[2-(N-methyl-3,4-dichlorophenylacetamido)-2-(3-guanidinophenyl)-ethyl]pyrrolidine
and the salts thereof, especially the pharmaceutically acceptable salts thereof.

Particular sub-groups of the compounds of the present invention of interest may be obtained by taking any one of the abovementioned particular generic definitions for R, $R^1$, $R^2$, $R^3$, $R^4$, $R^5$, $R^7$, $R^8$, $R^9$, $R^{10}$, $R^{11}$, $R^{12}$, M, W, $W^1$, $W^2$, $W^3$, X, Y or $Y^1$ either singly or in combination with any other particular generic definition(s) for R, $R^1$, $R^2$, $R^3$, $R^4$, $R^5$, $R^7$, $R^8$, $R^9$, $R^{10}$, $R^{11}$, $R^{12}$, M, W, $W^1$, $W^2$, $W^3$, X, Y or $Y^1$.

According to a further feature of the present invention there is provided a process for the preparation of a compound of the invention wherein $R^3$ is as hereinbefore defined with the proviso that the radical comprising an organic base, an organic acid or a quaternary ammonium salt is bonded to phenyl via oxygen or sulphur which process comprises reacting a compound corresponding to a compound of formula I but in which $R^3$ represents a meta hydroxyphenyl or mercaptophenyl group optionally further substituted as hereinbefore defined or a racemate thereof with a compound of the formula $R^6Z$ in which Z represents a leaving atom or group and $R^6$ represents a moiety containing an organic base, an organic acid or a quaternary ammonium salt, $R^6$ optionally being in protected form;

whereafter

(i) any protecting group present is removed to form a compound of the invention; and/or

(ii) where a compound of formula I or a racemate thereof is obtained in the form of a base or acid and a salt is required, reacting said compound of formula I or racemate thereof with respectively an acid or base to form a salt.

The reaction may for example be effected in the presence of a polar solvent, for example a polar aprotic solvent such as dimethylformamide. The reaction is also conveniently effected in the presence of an acid binding agent such as an alkali metal or alkaline earth metal carbonate or bicarbonate such as potassium, sodium or magnesium carbonate. The reaction may for example be effected at a temperature from 10 °C to the boiling temperature of the reaction mixture such as ambient temperature.

According to a further feature of the present invention there is provided a process for the preparation of a compound of the invention wherein $R^3$ is as hereinbefore defined with the proviso that the substituent in the meta-position comprises a quaternary ammonium salt and is bonded to phenyl via oxygen, -NH- or sulphur, which process comprises quaternising a compound which corresponds to a compound of formula I

but in which $R^3$ represents a group containing a tertiary amine.

The quaternisation may conveniently be effected in the presence of an alkyl or aralkyl halide such as chloride, bromide or iodide for example iodomethane or benzyl chloride and advantageously in the presence of a solvent inert under the reaction conditions. The quaternisation is also conveniently effected at a temperature of from 10°C to the reflux temperature of the reaction mixture, preferably at about ambient temperature. The quaternisation may for example be effected in the presence of a polar aprotic solvent such as acetone.

According to a further feature of the present invention there is provided a process for the preparation of a compound of the invention wherein $R^3$ is as hereinbefore defined with the proviso that the substituent in the meta-position is bonded to phenyl via -NH-, which process comprises reacting a compound corresponding to a compound of the formula I but in which $R^3$ represents a meta-aminophenyl group (optionally further substituted as hereinbefore defined) or racemate thereof with a compound of the formula $R^6Z$ (wherein $R^6$ is as hereinefore defined and Z represents a leaving atom or group or a cyano group), $R^6$ optionally being in protected form;

whereafter

(i) any protecting group present is removed to form a compound of the invention; and/or

(ii) where a compound of formula I or a racemate thereof is obtained in the form of a base or acid and a salt is required, reacting said compound of formula I or racemate thereof with respectively an acid or base to form a salt.

The reaction is conveniently effected at a temperature of from 0°C to the boiling temperature of the reaction mixture, preferably at a temperature of from 0°C to 25°C. The reaction may for example be effected in the presence of a polar solvent such as a $C_{1-4}$ alkanol e.g. ethanol.

According to a further feature of the present invention there is provided a process for preparing a compound of the invention, which comprises reacting a compound of formula IX (as set out hereinafter in which $R^1$ and X are as hereinbefore defined and $Y^2$ represents an acid or an activated derivative thereof) with a compound of formula X (as set out hereinafter in which $R^2$, $R^3$, $R^4$ and $R^5$ are as hereinbefore defined) or a racemate thereof, optionally in protected form, and where necessary deprotecting the compound thus obtained to form a compound of formula I or racemate thereof and if desired reacting said compound or racemate with an acid to form a salt.

Such a process may be employed to prepare the compounds corresponding to compounds of formula I but in which $R^3$ represents a meta-hydroxyphenyl or meta-aminophenyl group, such phenyl group being optionally further substituted as hereinbefore defined.

A compound of formula IX may for example be used in which $Y^2$ represents an ester or an acid halide such as an acid chloride, acid anhydride or acyl imidazole.

Where the compound of formula IX used is an ester the reaction may advantageously be effected in the presence of an aprotic solvent or in the absence of a solvent. Such a reaction is preferably effected at a temperature of from ambient to the reflux temperature of the reaction mixture, but where no solvent is employed the reaction is preferably effected at a temperature no greater than 150°C.

Where the compound of formula IX used is an acid chloride, an acid anhydride or an acyl imidazole the reaction is advantageously effected in the presence of an aprotic solvent, preferably at a temperature of from 0-60°C.

The compound of formula X may for example be prepared by reaction of a compound of formula XI (wherein p represents a hydrogen atom or an amine protecting group and $R^4$ and $R^5$ are as hereinbefore defined) to convert said compound into a compound of formula X.

Such conversion may be effected in a number of different ways. Thus for example a compound of formula X in which $R^2$ is hydrogen may be prepared by reducing a compound of formula XI and where P in the compound of formula XI represents an amine protecting group, if necessary,subjecting the reduced compound obtained to deprotection. Alternatively where a compound of formula XI is used in which P represents an amine protecting group, the compound of formula XI may be subjected to deprotection prior to reduction.

Where it is desired to prepare a compound of formula X in which $R^2$ represents an alkyl group, the said compound may for example be prepared by reduction before or after alkylation or where alkylation is effected by reductive alkanoylation simultaneously with reduction. Alternatively where a compound of formula XI is used in which P represents an appropriate amine protecting group said compound may be reacted to convert the protecting group into the desired alkyl group.

Thus where it is desired to prepare a compound formula X in which $R^2$ is methyl, a compound of formula X in which $R^2$ is hydrogen may be subjected to methylation whereby to obtain the desired compound. Such a compound of formula X in which $R^2$ is methyl may also be prepared by reacting a

compound of formula XI in which P represents an appropriate amine protecting group whereby to convert said amine protecting group into a methyl group. Such a reaction may for example be effected using a compound of formula XI in which P represents a benzyloxycarbonyl group, the reaction being effected by the use of a complex aluminium hydride reducing agent such as lithium aluminium hydride.

Where it is desired to prepare a compound of formula X in which $R^2$ is ethyl, n-propyl or isopropyl, a compound of formula X in which $R^2$ is hydrogen may be reacted with acetic or propionic acid or a reactive derivative thereof or with acetone, reduction being effected whereby to obtain the desired compound of formula X in which $R^2$ is ethyl, n-propyl or isopropyl.

The compound of formula XI may for example be prepared by reaction of a compound of formula XII (as set out hereinafter in which P and $Y^2$ are as hereinbefore defined) with a compound of formula XIII (as set out hereinafter in which $R^4$ and $R^5$ are as hereinbefore defined).

A compound of formula X may also be prepared by reduction of a compound of formula XIV in which $R^2$, $R^4$ and $R^5$ are as hereinbefore defined preferably using a complex aluminium hydride reducing agent such as lithium aluminium hydride. The compound of formula XIV is conveniently prepared by deprotecting a compound of formula XV (in which $P^1$ represents an amine protecting group and $R^2$, $R^4$ and $R^5$ are as hereinbefore defined), by methods known per se. The compound of formula XV may be prepared by reacting a compound of formula XVI (in which $R^1$, $R^2$ and Y are as hereinbefore defined) with a compound of formula XIII. The compound of formula XVI may be prepared by the amine protection of a compound of formula XVII.

The compounds corresponding to the compounds of the invention, but in which $R^3$ represents a phenyl group meta-substituted by hydroxy or amino and optionally further substituted as hereinbefore defined may also be prepared by the hydrolysis of a corresponding compound in which the said phenyl group is meta substituted by an acyloxy or acylamino group, or by the deprotection of a corresponding compound in which the said phenyl group is meta substituted by an alkoxy or benzyl group using, for example strong acid or boron tribromide.

If desired one compound of the present invention may be converted into another compound of the present invention by methods known per se.

Compounds of the invention and compounds corresponding to compounds of the invention but in which $R^3$ represents a phenyl group meta-substituted by hydroxy or amino and optionally further substituted as hereinbefore defined in which X represents a $-SOCH_2-$ moiety, may be prepared by oxidising a corresponding compound of formulae II or III in which X represents an $-SCH_2-$ or $-SOCH_2-$moiety.

Compounds of the invention and compounds corresponding to compounds of the invention but in which $R^3$ represents a phenyl group meta-substituted by hydroxy or amino and optionally further substituted as hereinbefore defined in which X represents an $-SOCH_2-$ moiety, may be prepared by oxidising a corresponding compound of formula II or III in which X represents an $-SCH_2-$ moiety.

The oxidation of -S- or -SO- to $-SO_2$ and the oxidation of -S-to -SO- is conveniently effected by methods known per se for example by the use of hydrogen peroxide advantageously in the presence of acetic acid or a haloacetic acid for example trifluoroacetic acid, or a peroxy acid, for example 3-chloroperbenzoic acid, advantageously in the presence of an appropriate chlorinated solvent, for example dichloromethane.

Where a 3-nitrophenylglycine is used as starting material the nitrophenyl moiety may if desired by reduced to the corresponding 3-aminophenyl moiety at any stage during the preparation of the compounds of the invention.

The compounds used as starting materials in the above-mentioned processes may contain radicals which are not compatible with at least certain of the processes described as will readily be appreciated by those skilled in the art and in such cases it is desirable to protect the starting material(s) by the use of an appropriate protecting group prior to the relevant reaction. After completion of the reaction the protecting group may if desired be removed or if desired may be left intact for removal after formation of the compound of formula I or racemate in protected form.

The relevant starting material may be in racemic form or in the desired optically active form. Where a racemate of the compound of formula I is obtained, the desired optically active enantiomer may be obtained by resolution according to conventional techniques. Where an optically active enantiomer of the compounds of formula I is obtained a racemate may if desired be obtained by racemisation according to conventional techniques.

Salts of the compounds of formula I or racemates thereof may if desired be prepared by reaction of the compound of formula I or racemate thereof with an appropriate acid, preferably an acid which affords a pharmaceutically acceptable anion for example a mineral acid such as hydrochloric acid or an organic acid such as fumaric or maleic acid.

Salts of the compound of formula I or racemate thereof may be converted to compounds of formula I or racemate thereof per se by conventional techniques, for example by ion exchange.

The compounds of the present invention are of interest in the treatment of medical conditions where stimulation of kappa receptors is appropriate, but where it is desired to avoid CNS (central nervous system) side effects. In particular compounds of the present invention possess hypotensive activity and certain compounds of the invention may possess an antiinflammatory activity.

The hypotensive activity of compounds of the invention has been demonstrated in the cardiovascular conscious dog test which was conducted as follows:-

Conscious male beagle dogs (14-18 kgs), with previously implanted chronic indwelling arterial and venous catheters are used in this test. The animals are trained to lie quietly in a padded box. At the beginning of each experiment, control blood pressure, heart rate, E.C.G and cardiac force measurements are taken. Aortic blood pressure is recorded via a pressure transducer and displayed on a direct writing oscillograph (MX4 or M19). Heart rate is electronically derived from the pulse pressure and displayed continuously. The ECG is derived via attached limb leads (lead II). Cardiac force measurements are made using the non-invasive technique of determination of the Q-A interval. After control measurements have been taken, the test substance is administered subcutaneously in a single dose. Subsequently at 2, 3 and 5 hours. In addition, any observable side effects will be noted.

The test substance was administered in a dose of 1.0 mg/kg dissolved in a small volume of 1% sodium bicarbonate and diluted with saline, the dose vehicle being 40% polyethylene alcohol/10% ethanol/40% saline and the dose volume being 5-10 mls.

Significant changes in blood pressure, when compared with controls, were noted. Standard controls used were clonidine-250 µg/kg po and hydrallazine-10 mg/kg po.

The compounds of the present invention may also find use as a research tool and thus may be useful in the delineation of the role of peripheral, as opposed to central nervous system, kappa receptors.

According to a further feature of the present invention therefore we provide pharmaceutical compositions comprising as active ingredient at least one compound of formula I or racemate thereof or a pharmaceutically acceptable salt of said compound or racemate in association with a pharmaceutically acceptable carrier or diluent.

The pharmaceutical compositions of the invention may be in a form suitable for oral, parenteral, topical or rectal administration. Thus, for example, they may be in orally-administrable unit dosage form, for example tablets or capsules, which may optionally be adapted for sustained release, or in injectable form, for example a sterile injectable solution or suspension, or in the form of a suppository for rectal administration or in a topically administrable form for antiinflamatory indications, for example an ointment or a nasal spray. The said pharmaceutical compositions may be produced by conventional methods using conventional diluents or carriers.

The compositions of the present invention may have veterinary as well as human applications, but where one of the compounds of the invention is used clinically in humans it is recommended to employ doses from 10 ug to 300 mg, for example 0.1 to 50 mg, once to four times per day, such a dosage range encompassing all routes of administration. No overt toxicity was noted at physiologically active doses.

According to a further feature of the present invention we provide a method of alleviating hypertension and or inflammation in warm-blooded animals which comprises administering to such animals an effective amount of a compound of formula I or a racemate or pharmaceutically acceptable salt of said compound or racemate.

The invention is illustrated but not limited by the following Examples in which the temperatures are expressed in degrees Celsius:-

## EXAMPLE 1

2(R,S)-N-[2-(N-methyl-3,4-dichlorophenylacetamido)-2-(3-carboxymethoxyphenyl)-ethyl]pyrrolidine hydrochloride.

2(R,S)-N-[2-(N-methyl-3,4-dichlorophenylacetamido)-2-(3-hydroxyphenyl)-ethyl]pyrrolidine (29.56 g), anhydrous potassium carbonate (32.28 g) and dry DMF (400 ml) were stirred under argon and treated with tert-butyl bromoacetate (9.2 ml). The mixture was stirred for 18 hours at ambient temperature and then diluted with water (2.5 l) and the product extracted with ethyl acetate (500 ml). The organic phase was washed with 3 x 100 ml brine, dried over magnesium sulphate and evaporated to a yellow oil which was purified by flash chromatography on silica using 2% tert butanol in dichloromethane giving 17.1 g pale yellow oil which was the tert butyl ester of the desired product.

10.22 g of the above ester were heated and stirred with 100 ml 6N HCl on a steam bath over 1 3/4 hours. The mixture was then cooled and the white product filtered off and dried over 18 hours under vacuum over phosphorus pentoxide. This product was recrystallised from acetonitrile (3700 ml) and dried at 60° under vacuum for 18 hours to give the product as fine white crystals. Melting point 207-8°, yield 7.7 g.

2(R,S)-N-[2-(N-methyl-3,4-dichlorophenylacetamido)-2-(3-hydroxyphenyl)-ethyl]pyrrolidine used as starting material was obtained as follows:-

a) R,S-N-Benzyloxycarbonyl-2-(3-methoxyphenyl)glycine.

R,S-2-(3-methoxyphenyl)glycine (21.72 g) was stirred in 120 ml 2N sodium hydroxide at 10°C and 23.0 ml benzyl chloroformate added over 1/2 hour. After stirring for a further 1 hour the mixture was washed with ether, acidified, and the resulting oil extracted with ether. This ether solution was washed with water, dried ($MgSO_4$) and evaporated to give 38.7 g of a pale yellow oil which crystallised on stirring with hexane. The pale cream crystals were collected, washed, and dried with 32.0 g (85%), M.Pt 97.0°C.

b) R,S-N-Benzyloxycarbonyl-2-(3-methoxyphenyl)glycine, pyrrolidide.

31.5 g of R,S-2-(3-methoxyphenyl)glycine in 300 ml ethyl acetate was cooled in an ice/water bath and treated with 17.8 g carbonyldiimidazole. After stirring for 2 hours at 10-15°C, 92 ml of pyrrolidine were added (mild exotherm). The mixture was stirred for 1 hour, washed three times with 2N HCl, once with saturated aq.$NaHCO_3$, and water, dried over $MgSO_4$ (+ decolourising charcoal), and evaporated to give 36.1 g (98%) of a very pale green oil.
Mass spectrum M/e 369 $(M + H)^+$, NMR (deuterochloroform): δ1.8 (4H,m), δ3.4 (4H,m), δ3.8 (3H,s), δ4.8 (2H,d), δ5.3 (1H,s), δ6.3 (1H,d) δ6.9 and 7.3 (9H,m).

c) R,S-1-(3-methoxyphenyl)-1-methylamino-2-(1-pyrrolidino)ethane.

11.4 g lithium tetrahydroaluminate was stirred in 500 ml dry THF under argon and 36.0 g R,S-N-Benzyloxycarbonyl-2-(3-methoxyphenyl)glycine, pyrrolidide was added over 1/2 hour at 10-15°C. The mixture was stirred for 1/2 hour, and then heated 2 hours at 55°C. After cooling in ice the mixture was carefully quenched with excess saturated aqueous sodium carbonate solution, filtered, and evaporated to give 36 g colourless oil which was used without further purification.

d) R,S-1-(3,4-dichloro-N-methylacetamido)-1-(3-methoxyphenyl)-2-(1-pyrrolidino) ethane.

The above oil was dissolved in 250 ml dichloromethane and 26.8 g 3,4-dichlorophenylacetylchloride was added. After 10 minutes 600 ml ether were added and the mixture stood 18 hours at ambient temperature. The product (white crystals) was collected, washed with ether, and dried -wt 3.5 g (73% yield).

1.1 g of this product recrystallised from ethyl acetate containing a trace of methanol gave 850 mg pure product M.Pt 221-2°C.

e) R,S-1-(3,4-dichloro-N-methylacetamido)-1-(3-hydroxyphenyl)-2-(1-pyrrolidino)-ethane.

A solution of 44.0 g of the above methyl ether in 290 ml dichloromethane were stirred at -72°C and 290 ml 1M. Boron tribromide in dichloromethane were added. The mixture was stirred for 1 hr at -70°C, warmed to ambient temperature and stirred for 6 hours. The mixture was carefully quenched at 0°C with 500 ml methanol and the solution evaporated under reduced pressure. The resultant solid was stirred overnight with methanol/ether (200 ml and 300 ml) and filtered to give the product as a white solid (30.6 g) which was a mixture of the hydrochloride and hydrobromide salts of the products (this mixture being confirmed by acid/base filtration).
NMR (deutero DMSO + deuteroacetic acid) δ2.1 (4H,m), 2.7 (3H,s), δ3.1-4.2 (8H,m), δ6.1 (1H,9), δ6.7-7.5 (7H,m).
Mass spectrum $(M + H)^+$ 407 under +ve FAB conditions.

3,4-Dichlorophenylacetyl chloride

3,4-Dichlorophenylacetic acid (76.8 g) was dissolved in dry dichloromethane (300 ml) and oxalyl chloride (52 g, 36 ml) was added to the solution obtained in one portion. The reaction mixture was protected

from atmospheric moisture with a calcium chloride guard tube and stirred mechanically whilst dimethylformamide (3 drops) was added. The solution was stirred at ambient temperature for 18 hours and then evaporated under reduced pressure to give a yellow oil. Distillation in vacuo afforded 80.0 g of 3,4-dichlorophenylacetyl chloride, b.pt 97-100°/0.6 mm, as a viscous pale yellow oil.

## EXAMPLE 2

(R,S)-N-[2-(N-methyl-3,4-dichlorophenylacetamido)-2-(3-trimethylammoniumpropoxyphenyl)-ethyl]pyrrolidine iodide hydrochloride.

1.0 g of (R,S)-N-[2-(N-methyl-3,4-dichlorophenylacetamido)-2-(3-dimethylaminopropoxyphenyl)ethyl]-pyrrolidine in 5 ml acetone (analytical grade) was treated with 0.29 g iodomethane. After 72 hours at ambient temperature white crystals had separated and the mixture was filtered, the solid rejected, and the filtrate evaporated to give an oil which crystallised on stirring with ether. This (hygroscopic) solid was dried and recrystallised from acetone/ether (again rather hygroscopic). Stirred with ether and dried to give the product (500 mg) M.Pt 50-3°.

The starting material (R,S)-N-[2-(N-methyl-3,4-dichlorophenylacetamido)-2-[3-dimethylaminopropoxy)-phenyl]-ethyl]pyrrolidine was obtained as follows:-
3.46 g of (R,S)-N-[2-(N-methyl-3,4-dichlorophenylacetamido)-2-(3-hydroxyphenyl)-ethyl]pyrrolidine in 20 ml toluene were treated with 0.375 g of 55% sodium hydride in mineral oil (under Argon atmosphere) and the mixture stirred at 80°. 8.0 g Dimethylaminopropyl chloride hydrochloride were dissolved in 2 ml water and excess sodium hydroxide solution (10 molar) was added. The solution was extracted with 3 x 12 ml toluene and the extracts (not washed) dried over potassium hydroxide pellets and diluted to 50 ml with toluene. 25 ml of this solution were added over one hour to the phenoxide solution, and 10 mg sodium iodide added. The reaction was slow and so a further 25 ml of the halide solution was added and the reaction heated for 16 hours at 80°. After dilution with ethyl acetate the mixture was washed with water, dried over magnesium sulphate, and evaporated to give 5.0 g of pale yellow oil. This was dissolved in ether, treated with charcoal for a few minutes, filtered and treated with a solution of hydrogen chloride in ethyl acetate. The solid produced, which was in the form of a gum, was washed with ether and recrystallised from isopropanol to give 3.2 g of a white crystalline solid. 700 mg of this solid were recrystallised from ethyl acetate containing a few drops of methanol to give the pure dihydrochloride monohydrate of (R,S)-N-[2-(N-methyl-3,4-dichlorophenylacetamido)-2-(3-dimethylaminopropoxyphenyl)-ethyl]pyrrolidine m.p. 174-5°.

The compound (R,S)-N-[2-(N-methyl-3,4-dichlorophenylacetamido)-2-(3-hydroxyphenyl)-ethyl]pyrrolidine used as starting material herein was obtained as described in Example 1.

## EXAMPLE 3

(R,S)-N-[2-(N-methyl-3,4-dichlorophenylacetamido)-2-(3-benzyldimethylammoniumpropoxyphenyl)-ethyl-pyrrolidine chloride hydrochloride dihydrate.

This compound was made in an analogous manner to Example 2, using benzyl chloride in place of iodomethane. The product was not crystalline, evaporation of the acetone solution yielding a stable foam.

| Analysis | | | | | |
|---|---|---|---|---|---|
| | C | H | N | $Cl^-$ | Water |
| Found | 61.2 | 6.8 | 6.3 | 5.1 | 5.1 |
| Theory | 60.7 | 6.8 | 6.4 | 5/4 | 5.5 |

NMR (deuterochloroform) δ1.75 (4H, broad s), δ2.2-2.6 (6H,m), δ2.7 (5H,t), δ2.9-3.4 (1H,m), δ3.35 (6H,s), δ3.75 (4H, m), δ4.0 (2H,m), δ5.0 (2H,s), δ6.0 (1H,q), δ6.7-7.7 (12H,m).
Mass Spectrum m/e 582 (+ me FAB conditions).

## EXAMPLE 4

R,S)-N-[2-(N-methyl-3,4-dichlorophenylacetamido)-2-(3-guanidinophenyl)-ethyl]pyrrolidine dihydrate.

(R,S)-N-[2-(N-Methyl-3,4-dichlorophenylacetamido)-2-(3-aminophenyl)-ethyl]pyrrolidine dihydrochloride hemihydrate (400 mg) and cyanamide (100 mg) were heated together in ethanol (10 ml) at reflux for 48 hours. During this time the ethanol was slowly allowed to evaporate off. The residue was chromatographed on silica 7734 (E.Merck) using $CHCl_3$:MeOH:$NH_3$ (60:40:5) parts by volume to give a white foam.

This was purified by preparative layer chromatography on 1.5 mm thick alumina prep.plates using 20% methanol/ methylene chloride. The alumina/product spot was scraped off and extracted with methanol. This was evaporated and re-extracted into methylene choride. On filtration and evaporation the product was obtained as a white foam 86 mgs (18% yield).

| Analysis | | | | |
|---|---|---|---|---|
| | C | H | N | Water |
| Found | 54.6 | 6.1 | 15.2 | 7.3 |
| Theory | 54.5 | 6.4 | 14.5 | 7.4 |

for $C_{22}H_{27}N_5Cl0.2H_2O$ (484)
Mass spec. Molec. ion 448 $(M + H)^+$
NMR. $H^1$NMR (DMSO $d_6$) 2.0 ppm. ($CH_2$ x 2 4H broad) 2.88 ($NCH_3$ 3H singlet) 3.0-3.22 ($CH_2$N 2H broad) 3.45-4.1 ($CH_2$N X 2 4H broad) 3.75-4.2 (Ar$CH_2$CO 4H doublet of doublets) 6.12 CHAr (1H doublet of doublets) 7.1-7.7 (ArH broad multiplet).

The starting material (R,S)-N-[2-(N-methyl-3,4-dichlorophenylacetamido)-2-(3-aminophenyl)-ethyl]-pyrrolidine was obtained as follows:-

(a) (R,S)-N-[2-methoxycarbonylamino-2-(3-aminophenyl)acetyl] pyrrolidine.

N-Methoxycarbonyl-(R,S)-3-nitrophenylglycine pyrrolidide (15.0 g) [prepared by protecting 3-nitrophenyl-glycine in conventional manner and subsequent reaction of protected compound with pyrrolidine in similar manner to that described in Example 1a] was dissolved in glacial acetic acid (200 ml). 3-nitrophenylglycine may be prepared by the method of Sriid and Kjaer, Acta. Chim.Scand. 1963, 17, page 2394.

The catalyst (10% Pd/C) (2.0 g) was added and the mixture was stirred at room temperature under a hydrogen atmosphere until uptake of the gas was complete. The hydrogen atmosphere was flushed away with argon and the mixture filtered through Celite to give a clear solution. This was evaporated and then azeotroped several times with toluene to remove the last traces of acetic acid to give an oil which slowly crystallised (14.5 g) m.p. 147-9° (isopropanol).

(b) (R,S)-N-[2-methylamino-2-(3-aminophenyl)ethyl] pyrrolidine.

The (R,S)-N-[2-methoxycarbonylamino-2-(3-aminophenyl)acetyl]pyrrolidine (5.0 g) was dissolved in dry tetrahydrofuran (100 ml) and added to a suspension of lithium aluminium hydride (2.0 g) in tetrahydrofuran (50 ml) with stirring whilst cooling in ice and under an argon atmosphere. After completion of the addition the mixture was stirred at reflux for 16 hours. After cooling to room temperature the excess lithium aluminum hydride was destroyed using saturated sodium carbonate solution, and the mixture was filtered and evaporated to leave an oil 3.5 g. This oil was columned using 30% methanol 70% methylene chloride and 0.1% aqueous ammonia (S.G. 0.88) on Merck silica 7734 to give an oil 2.7 g as the product (68% yield).

Mass Spec. molecular ion at 220 $(M + H)^+$ NMR: $H^1$ NMR ($CD_3CO_2D$) 2.0 ppm ($CH_2$x2 4H broad) 2.55 ($NCH_3$ 3H singlet) 3.35-4.4 (one $CH_2$ masked by water from the $CD_3CO_2D$) ($CH_2$Nx3 6H multiplet) 4.75 (CH 1H triplet) 6.85-7.5 (CH aromatic x 4 4H multiplet).

(c) (R,S)-N-[2-(N-methyl-3,4-dichlorophenylacetamido)-2-(3,4-dichlorophenylacetamido)-phenyl-ethyl]-pyrrolidine.

(R,S)-N-[2-methylamino-2-(3-aminophenyl)ethyl]-pyrrolidine (14.3 g) was dissolved in dichloromethane (1200 ml) and 3,4-dichlorophenylacetyl chloride (29.2 g) was added slowly with ice cooling to the stirred solution. After completion of the addition the mixture was stirred for 3 hours at room temperature. The solvent was evaporated and the residue was triturated with ether and filtrated to give an off white solid 34.0 g m.p. 249-251° (methanol/ethyl acetate).

(d) (R,S)-N-[2-(N-Methyl-3,4-dichlorophenylacetamido)-2-(3-aminophenyl)ethyl]pyrrolidine hydrochloride.

(R,S)-N-[2-(N-Methyl-3,4-dichlorophenylacetamido)-2-(3,4-dichlorophenylacetamidophenyl)ethyl] pyrrolidine (34 g) was refluxed in 20: 80 water: ethanol (by volume) (200 ml) containing sodium hydroxide pellets (20 g) overnight. The mixture was evaporated under reduced pressure to remove most of the ethanol and then extracted with ethyl acetate. The organic layer was washed with water and then brine and finally dried ($MgSO_4$) and evaporated to give an oil, yield: 21.0 g.

A sample with ethereal hydrogen chloride gave a white solid (dihydrochloride) m.p. 248-9°. (methanol/ethyl acetate).

The Octan-1-ol-water distribution coefficient referred to hereinbefore may be determined as follows:-

The compound for test is dissolved (1 mg/50 ml) in buffer (0.01M Phosphate, pH 7.4) presaturated with the organic solvent. The u.v. spectrum is recorded to ensure that the u.v. absorbance is between 0.3 and 1.5 units for at least one band in the spectrum, preferably at $\lambda > 250$ nm. The solution is filtered to remove excess solid material and transferred (5 ml) by pipette into a stoppered test tube. The required volume of octan-1-ol, presaturated with water at the appropriate pH, is then transferred, again by pipette into the test tube. Ideally, the ratio of aqueous to organic should be such that an absorbance change of fifty per cent is achieved, although a minimum aqueous volume of 3 ml is required. The stoppered test tube is then vigorously shaken for several minutes and allowed to stand for fifteen minutes. The mixture is transferred by pipette into a centrifuge tube and placed in the centrifuge opposite a second tube containing an equal volume of water and spun at 3600 r.p.m. for ten minutes. The centrifuge tube is removed and the upper layer is carefully removed by pasteur pipette. A second centrifugation may be necessary after this process to reseparate the layers. Using a second, clean pasteur pipette placed to the bottom of the centrifuge tube with application of positive pressure, the aqueous layer (2-3 ml) is transferred to the cuvette taking care not to carry over droplets of the organic layer. The u.v.spectrum of this solution is taken overlaid onto the u.v. spectrum of the starting aqueous stock solution, both referenced against aqueous buffer. The spectrum of the stock solution should be compared with the original spectrum to ensure no degradation of the compound has occurred during the experiment.

The distribution coefficient, D, is calculated using the following equation -

$$D = \frac{A_{B,w} - A_{A,w}}{A_{A,w}} \times \frac{V_w}{V_o}$$

$A_{B,w}$ = Absorbance in aqueous phase before addition of organic layer
$A_{A,w}$ = Absorbance in aqueous phase after addition of organic layer
$V_w$ = Volume of the aqueous phase
$V_o$ = Volume of organic phase

The result must be the same whatever the wavelength chosen. In all cases the measurement is confirmed by repeating the experiment by partitioning from the organic solvent into water. The result of the reverse experiment can be calculated by the following expression -

$$D = \frac{A_{A,o}}{A_{B,o} - A_{A,o}} \times \frac{V_w}{V_o}$$

$A_{A,o}$ = Absorbance in organic phase before addition of buffer
$A_{B,o}$ = Absorbance in organic phase after addition of buffer.

## Pharmaceutical Composition Examples

### Example A

Tablets:

Each tablet contains:

| | |
|---|---|
| 2(RS)-N-[2-(N-methyl-3,4-dichlorophenylacetamido)-2-(3-carboxymethoxyphenyl-ethyl]pyrrolidine hydrochloride | 0.1mg |
| Dicalcium phosphate | 42mg |
| Methylcellulose, U.S.P (15 c.p.s) | 1.6mg |
| Lactose | 6mg |
| Calcium Stearate | 0.5mg |

The active ingredient and dicalcium phosphate are mixed well, granulated with a 7.5% aqueous solution of methylcellulose, passed through a No. 8 screen and dried. The granulate obtained is then passed through a No. 12 screen, mixed with the remaining ingredients (lactose and calcium stearate) and compressed to yield tablets each weighing approximately 50mg.

### Example B

Capsules:

Each capsule contains:

| | |
|---|---|
| Compound of the present invention | 0.2mg |
| Lactose U.S.P. | 47mg |
| Starch U.S.P | 2.5mg |
| Calcium stearate | 0.35mg |

The above-mentioned ingredients are obtained in finely powdered form, mixed thoroughly and then filled into appropriately sized hard gelatin capsules.

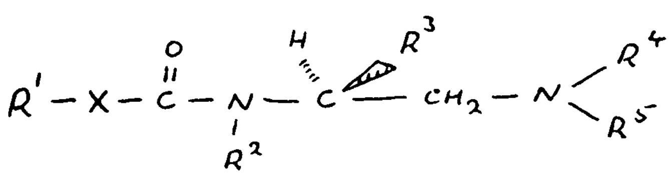

I

-Y-W-M II

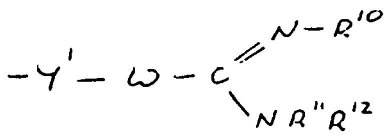

III

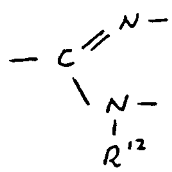

IV

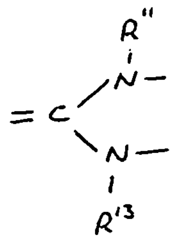

IVa

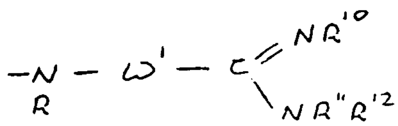

V

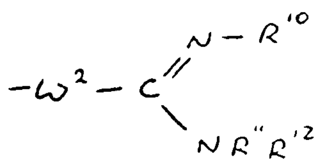

VI

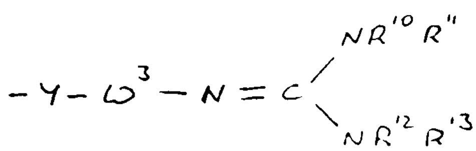

VII

$-Y-W^2-A$ VIII

$R^1$-X-$Y^2$ IX

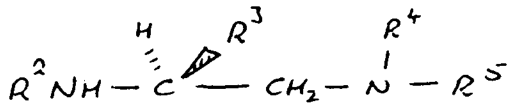

X

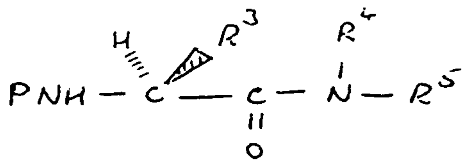

XI

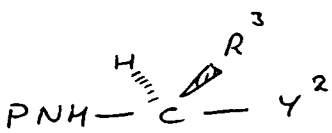

XII

$R^4$-NH-$R^5$ XIII

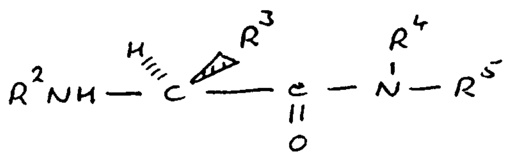

XIV

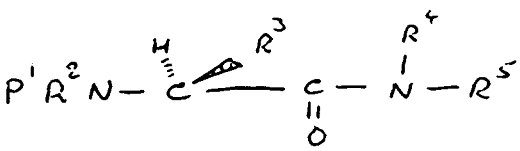

XV

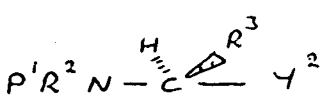

XVI

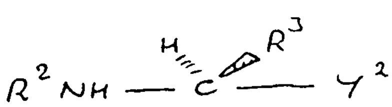

XVII

**Claims**

**Claims for the following Contracting States : BE, CH, DE, LI, NL**

**1.** A compound of formula I

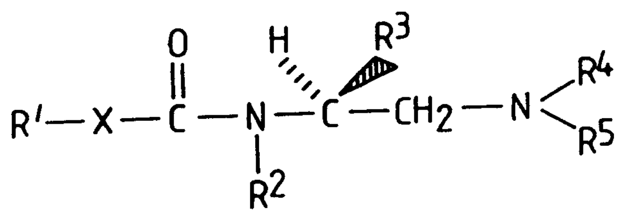

I

[wherein $R^1$ represents a halophenyl, dihalophenyl, nitrophenyl, cyanophenyl or trifluoromethylphenyl group;

X represents a single bond, $-CH_2-$, $OCH_2-$, $-SCH_2-$, $-S(0)-CH_2-$, $-S(0)_2-CH_2-$ or $-CH_2-CH_2-$;

$R^2$ represents hydrogen or $C_{1-3}$ alkyl;

$R^3$ represents a phenyl group substituted in the meta position by a radical containing an organic base, an organic acid or a quaternary ammonium salt and optionally further substituted by halogen, $C_{1-3}$alkyl, $C_{1-3}$alkoxy, cyano, nitro, hydroxy, amino, mono-or di-($C_1$ $_{or}$ $_2$alkyl)amino, carboxamido, mono- or di-($C_1$ $_{or}$ $_2$alkyl)carboxamido, ureido or $C_{1-3}$acylamino;

$R^4$ and $R^5$, which may be the same or different, each represents a $C_{3-5}$ alkenyl, $c_{3-5}$ alkyl, $C_{1-6}$ alkyl, or $C_{4-7}$ cycloalkylalkyl group;

or $R^4$ and $R^5$ together with the intervening nitrogen atom represent a 4-7-membered heterocyclic ring which optionally contains a further heteroatom selected from oxygen and sulphur] or racemates thereof, and the salts of said compounds or racemates; the group $-R^3$ being selected such that the compound of formula I as defined herein or racemate thereof or salt of said compound of formula I as defined herein or racemate thereof has a log D of less than 0.5 at pH 7.4 (where log D is the logarithm of the distribution coefficient between octan-1-ol and aqueous buffer).

**2.** A compound as claimed in claim 1 wherein $R^3$ represents phenyl substituted in the meta-position by:-

(i) a group of the formula;-

-Y-W-M II

in which Y represents a single bond, -S-, -0- or -NR- (in which R represents hydrogen, methyl, formyl or acetyl), W represents a $C_{2-4}$alkylene chain which may if desired be branched and M represents the group $-NR^7R^8R^9$ (wherein $R^7$, $R^8$ and $R^9$, which may be the same or different, each represents a $C_{1-7}$alkyl or aralkyl group;

(ii) a group of the formula III

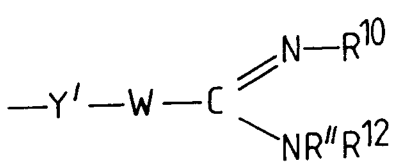

III

in which $Y^1$ represents -0- or -S-; W represents a $C_{2-4}$alkylene chain which may if desired be branched; and $R^{10}$, $R^{11}$ and $R^{12}$, which may be the same or different, each represents hydrogen or methyl or $R^{10}$ and $R^{11}$ together represent a $C_2$ or $C_3$ alkylene chain such that $R^{10}$ and $R^{11}$ together with the intervening radical of formula IV

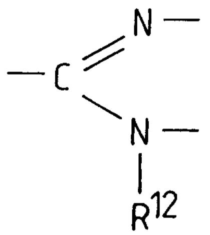

IV

form a 4,5-dihydroimidazol-2-yl or 3,4,5,6-tetrahydro-pyrimidin-2-yl ring, $R^{12}$ representing hydrogen or methyl; or $R^{11}$ and $R^{12}$ together represent a $C_4$ or $_5$ alkylene chain such that $R^{11}$ and $R^{12}$ together with the intervening nitrogen atom form a pyrrolidine or piperidine ring and $R^{10}$ represents hydrogen or methyl;
(iii) a group of the formula V

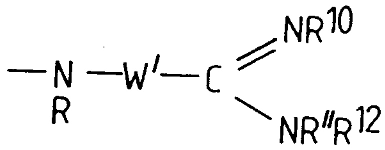

V

in which $W^1$ represents a $C_{2-4}$alkylene chain which may if desired be branched and $R^{10}$, $R^{11}$ and $R^{12}$ are as hereinbefore defined.
(iv) a group of the formula VI

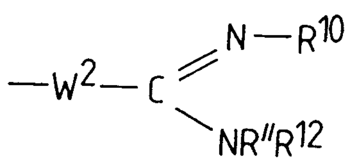

VI

in which $W^2$ represents a $C_{1-4}$ alkylene chain which may if desired be branched and $R^{10}$, $R^{11}$ and $R^{12}$, are as hereinbefore defined;
v) a group of the formula VII

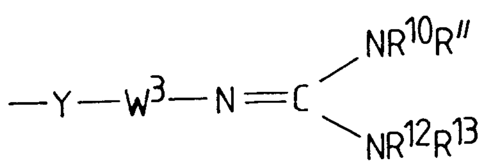

VII

in which Y is as hereinbefore defined, $W^3$ represents a $C_{2-4}$alkylene chain which may if desired be branched or where Y represents a single bond $W^3$ may represent a single bond or a $C_{1-4}$alkylene chain which may if desired be branched and $R^{10}$, $R^{11}$, $R^{12}$ and $R^{13}$, which may be the same or different, each represents hydrogen or methyl,

or $R^{10}$ and $R^{12}$ together represent a $C_2$ or $_3$ alkylene chain such that $R^{10}$ and $R^{12}$ together with the intervening radical of formula IVa

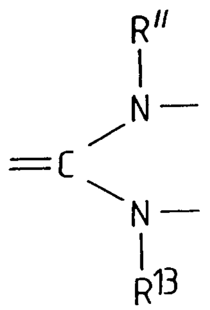

IVa

form a 4,5-dihydroimidazol-2-yl or 3,4,5,6-tetrahydropyrimidin-2-yl ring or tautomer thereof, $R^{11}$ and $R^{12}$ being independently selected from hydrogen or methyl,

or either $R^{10}$ and $R^{11}$ together or $R^{12}$ and $R^{13}$ together represent a $C_4$ or $_5$ alkylene chain such that either $R^{10}$ and $R^{11}$ together with the intervening nitrogen atom form a pyrrolidine or piperidine ring and $R^{12}$ and $R^{13}$ are independently selected from hydrogen and methyl or $R^{12}$ and $R^{13}$ together with the intervening nitrogen atom form a pyrrolidine or piperidine ring and $R^{10}$ and $R^{11}$ are independently selected from hydrogen and methyl; or

(vi) a group of the formula VIII

$-Y-W^2-A$ VIII

in which Y and $W^2$ are as hereinbefore defined and A represents a carboxylic or sulphonic acid.

**3.** A compound as claimed in Claim 1 or 2 wherein the organic base, the organic acid or the quaternary ammonium salt is linked to phenyl at the meta position via oxygen or -NH.

**4.** A compound as claimed in any one of the preceding claims wherein $R^4$ and $R^5$, which may be the same or different, are each a $C_{1-6}$alkyl group or a $C_{3-5}$alkenyl group, or $R^4$ and $R^5$ together with the intervening nitrogen atom represents a 4-7 membered ring optionally containing a further heteroatom.

**5.** A compound as claimed in any one of the preceding claims wherein $R^1$ represents chlorophenyl, dichlorophenyl, bromophenyl, fluorophenyl, difluorophenyl, trifluoromethylphenyl, nitrophenyl or cyanophenyl.

**6.** A compound as claimed in any one of the preceding claims wherein X stands for $-CH_2-$, $-OCH_2-$, $-SCH_2-$ or $-CH_2CH_2-$.

**7.** A compound as claimed in any one of the preceding claims wherein $R^3$ stands for phenyl substituted in the meta-position by:

i) a group of formula II as defined in claim 2 in which W is $C_2$ or $C_3$ alkylene;

ii) a group of formula III as defined in claim 2 in which W is $CH_2CH_2$;

iii) a group of formula V as defined in claim 2 in which $W^1$ is a single bond or $-CH_2CH_2-$;

iv) a group of formula VI as defined in claim 2 in which $W^2$ is $-CH_2-$ or $-CH_2CH_2-$;

v) a group of formula VII as defined in claim 2 in which Y represents -0-, -S- or -NR- (wherein R is as hereinbefore defined) and $W^3$ is $-CH_2CH_2-$; or a group of formula VII as defined in claim 2 in which Y represents a single bond and $W^3$ is a single bond - $CH_2$- or $CH_2CH_2$-; or

vi) A group of formula VIII as defined in claim 2 in which $W^2$ is $-CH_2-$.

**8.** A compound as claimed in claim 7 wherein when the grouping >N-R is present, as in formulae II, VII or VIII as defined in claim 2 in which Y represents >N-R, or in formula V as defined in claim 2, R is hydrogen.

**9.** A compound as claimed in claim 7 or 8 wherein when the groupings $R^7$, $R^8$ or $R^9$ are present, such groupings, which may be the same or different, are selected from methyl or benzyl.

**10.** A compound as claimed in any one of the preceeding claims wherein $R^3$ represents 3-(3-dimethylaminopropoxy)phenyl in the form of a quaternary ammonium salt, 3-(3-carboxymethoxy)phenyl, 3-guanidinophenyl; or a 3-trimethylammonium propoxy or 3-benzyldimethyl ammoniumpropoxy group stabilised by an appropriate anion.

**11.** A compound as claimed in any one of the preceding claims wherein $R^4$ and $R^5$ together with the intervening nitrogen atom represents a pyrrolidino, dimethylamino, diethylamino, N-allyl-N-methylamino, N-isopropyl-N-methylamino, $\Delta^3$-pyrrolino, or piperidino group.

**12.** A compound as claimed in claim 1 which comprises 2(R,S)-N-[2-(N-methyl-3,4-dichlorophenylacetamido)-2-(3-carboxymethoxyphenyl)-ethyl]pyrrolidine, (R,S)-N-[2-(N-methyl-3,4-dichlorophenylacetamido)-2-(3-trimethylammoniumpropoxyphenyl)-ethyl]-pyrrolidine iodide, (R,S)-N-[2-(N-methyl-3,4-dichlorophenylacetamido)-2-(3-benzyldimethylammoniumpropoxyphenyl)-ethyl]-pyrrolidine chloride; (R,S)-N-[2-(N-methyl-3,4-dichlorophenylacetamido)-2-(3-guanidinophenyl)-ethyl]pyrrolidine and the salts thereof.

**13.** A process for preparing a compound of formula I as defined in claim 1 or a salt thereof, which comprises:-

(a) for the preparation a compound of formula I as defined in claim 1, reacting a compound of formula IX

$R^1$-X-$Y^2$ IX

in which $R^1$ and X are as hereinbefore defined and $Y^2$ represents an acid or an activated derivative thereof) with a compound of formula X

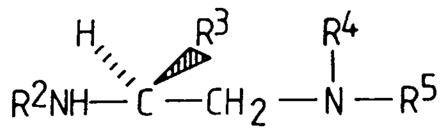

X

(as set out herinafter in which $R^2$, $R^3$, $R^4$ and $R^5$ are as hereinbefore defined) or a racemate thereof, optionally in protected form, and where necessary deprotecting the compound thus obtained to form a compound of formula I as defined in claim 1 or racemate thereof and if desired reacting said compound or racemate with an acid to form a salt;

(b) for the preparation of a compound of formula I as defined in claim 1 in which $R^3$ represents a phenyl group meta-substituted by hydroxy or amino and optionally further substituted as hereinbefore defined, hydrolysing a corresponding compound in which the said phenyl group is meta substituted by an acyloxy or acylamino group, or by the deprotection of a corresponding compound in which the said phenyl group is meta substituted by an alkoxy or benzyl group;

(c) for the preparation of a compound of formula I as defined in claim 1 in which $R^3$ represents a phenyl group meta-substituted by hydroxy or amino and optionally further substituted as hereinbefore defined in which X represents a $-SOCH_2$ moiety, oxidising a corresponding compound of formula II or III as defined in claim 2 in which X represents a $-SCH_2-$ or $-SOCH_2-$ moiety;

(d) for the preparation of compounds of formula I as defined in claim 1 in which $R^3$ represents a phenyl group meta-substituted by hydroxy or amino and optionally further substituted as hereinbefore defined in which X represents an $-SOCH_2-$ moiety, oxidising a corresponding compound of formula II or III as defined in claim 2 in which X represents a $-SCH_2-$ moiety;

(e) for the preparation of a compound of formula I as defined in claim 1 wherein $R^3$ is as hereinbefore defined with the proviso thatthe radical comprising an organic base, an organic acid or a quaternary ammonium salt is bonded to phenyl via oxygen or sulphur which process comprises reacting a compound corresponding to a compound of formula I as defined in claim 1 but in which $R^3$ represents a meta hydroxyphenyl or mercaptophenyl group optionally further substituted as hereinbefore defined or a racemate thereof with a compound of the formula $R^6Z$ in which Z represents a leaving atom or group and $R^6$ represents a moiety containing an organic base, an

organic acid or a quaternary ammonium salt, $R^6$ optionally being in protected form;
whereafter

(i) any protecting group present is removed to form a compound of the invention; and/or

(ii) where a compound of formula I as defined in claim 1 or a racemate thereof is obtained in the form of a base or acid and a salt is required, reacting said compound of formula I as defined in claim 1 or racemate thereof with respectively an acid or base to form a salt.

(f) for the preparation of a compound of formula I as defined in claim 1 wherein $R^3$ is as hereinbefore defined with the proviso that the substituent in the meta-position comprises a quaternary ammonium salt and is bonded to phenyl via oxygen, -NH- or sulphur, which process comprises quaternising a compound which corresponds to a compound of formula I as defined in claim 1 but in which $R^3$ represents a group containing a tertiary amine.

(g) for the preparation of a compound of formula I as defined in claim 1 wherein $R^3$ is as hereinbefore defined with the proviso that the substituent in the meta-position is bonded to phenyl via -NH-, which process comprises reacting a compound corresponding to a compound of the formula I as defined in claim 1 but in which $R^3$ represents a meta-aminophenyl group (optionally further substituted as hereinbefore defined) or racemate thereof with a compound of the formula $R^6Z$ (wherein $R^6$ is as hereinefore defined and Z represents a leaving atom or group or a cyano group), $R^6$ optionally being in protected form;
whereafter

(i) any protecting group present is removed to form a compound of the invention; and/or

(ii) where a compound of formula I as defined in claim 1 or a racemate thereof is obtained in the form of a base or acid and a salt is required, reacting said compound of formula I as defined in claim 1 or racemate thereof with respectively an acid or base to form a salt.

14. A pharmaceutical composition comprising as active ingredient at least one compound of formula I as defined in claim 1 or racemate thereof or a pharmaceutically acceptable salt of said compound or racemate in association with a pharmaceutically acceptable carrier or diluent.

**Claims for the following Contracting State : ES**

1. A process for preparing a compound of formula I

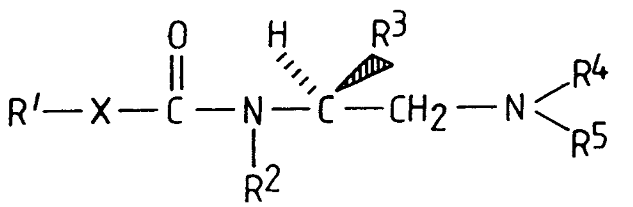

I

[wherein $R^1$ represents a halophenyl, dihalophenyl, nitrophenyl, cyanophenyl or trifluoromethylphenyl group;

X represents a single bond, $-CH_2-$, $OCH_2-$, $-SCH_2-$, $-S(0)-CH_2-$, $-S(0)_2-CH_2-$ or $-CH_2-CH_2-$;

$R^2$ represents hydrogen or $C_{1-3}$ alkyl;

$R^3$ represents a phenyl group substituted in the meta position by a radical containing an organic base, an organic acid or a quaternary ammonium salt and optionally further substituted by halogen, $C_{1-3}$alkyl, $C_{1-3}$alkoxy, cyano, nitro, hydroxy, amino, mono-or di-($C_{1\ or\ 2}$alkyl)amino, carboxamido, mono- or di-($C_{1\ or\ 2}$alkyl)carboxamido, ureido or $C_{1-3}$acylamino;

$R^4$ and $R^5$, which may be the same or different, each represents a $C_{3-5}$ alkenyl, $C_{3-5}$ alkynyl, $C_{1-6}$ alkyl, or $C_{4-7}$ cycloalkylalkyl group;

or $R^4$ and $R^5$ together with the intervening nitrogen atom represent a 4-7-membered heterocyclic ring which optionally contains a further heteroatom selected from oxygen and sulphur] or racemates thereof, and the salts of said compounds or racemates; the group $-R^3$ being selected such that the compound of formula I as defined herein or racemate thereof or salt of said compound of formula I as defined herein or racemate thereof has a log D of less than 0.5 at pH 7.4 (where log D is the logarithm of the distribution coefficient between octan-1-ol and aqueous buffer).

which comprises;

(a) for the preparation a compound of formula I, as defined herein reacting a compound of formula IX

$R^1$-X-$Y^2$ IX

in which $R^1$ and X are as hereinbefore defined and $Y^2$ represents an acid or an activated derivative thereof) with a compound of formula X

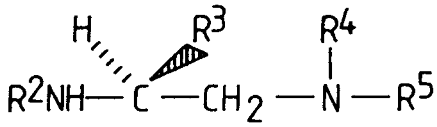

X

in which $R^2$, $R^3$, $R^4$ and $R^5$ are as hereinbefore defined) or a racemate thereof, optionally in protected form, and where necessary deprotecting the compound thus obtained to form a compound of formula I as defined herein or racemate thereof and if desired reacting said compound or racemate with an acid to form a salt;

(b) for the preparation of a compound of formula I as defined herein in which $R^3$ represents a phenyl group meta-substituted by hydroxy or amino and optionally further substituted as hereinbefore defined, hydrolysing a corresponding compound in which the said phenyl group is meta substituted by an acyloxy or acylamino group, or by the deprotection of a corresponding compound in which the said phenyl group is meta substituted by an alkoxy or benzyl group;

(c) for the preparation of a compound of formula I as defined herein in which $R^3$ represents a phenyl group meta-substituted by hydroxy or amino and optionally further substituted as hereinbefore defined in which X represents a -$SOCH_2$ moiety, oxidising a corresponding compound of formula II

-Y-W-M II

or III

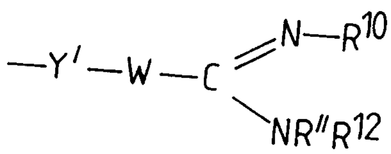

III

in which X represents a -$SCH_2$- or -$SOCH_2$- moiety;

(d) for the preparation of compounds of formula I as defined herein in which $R^3$ represents a phenyl group meta-substituted by hydroxy or amino and optionally further substituted as hereinbefore defined in which X represents an -$SOCH_2$ - moiety, oxidising a corresponding compound of formula II or III as defined herein in which X represents a -$SCH_2$ - moiety;

(e) for the preparation of a compound of formula I as defined herein wherein $R^3$ is as hereinbefore defined with the proviso that the radical comprising an organic base, an organic acid or a quaternary ammonium salt is bonded to phenyl via oxygen or sulphur which process comprises reacting a compound corresponding to a compound of formula I as defined herein but in which $R^3$ represents a meta hydroxyphenyl or mercaptophenyl group optionally further substituted as hereinbefore defined or a racemate thereof with a compound of the formula $R^6$Z in which Z represents a leaving atom or group and $R^6$ represents a moiety containing an organic base, an organic acid or a quaternary ammonium salt, $R^6$ optionally being in protected form;

whereafter

(i) any protecting group present is removed to form a compound of the invention; and/or

(ii) where a compound of formula I as defined herein or a racemate thereof is obtained in the form of a base or acid and a salt is required, reacting said compound of formula I as defined herein or racemate thereof with respectively an acid base to form a salt.

(f) for the preparation of a compound of formula I as defined herein wherein $R^3$ is as hereinbefore defined with the proviso that the substituent in the meta-position comprises a quaternary ammonium salt and is bonded to phenyl via oxygen, -NH- or sulphur, which process comprises quaternising a compound which corresponds to a compound of formula I as defined herein but in which $R^3$ represents a group containing a tertiary amine.

(g) for the preparation of a compound of formula I as defined herein wherein $R^3$ is as hereinbefore defined with the proviso that the substituent in the meta-position is bonded to phenyl via -NH-, which process comprises reacting a compound corresponding to a compound of the formula I as defined herein but in which $R^3$ represents a meta-aminophenyl group (optionally further substituted as hereinbefore defined) or racemate thereof with a compound of the formula $R^6Z$ as - 5-

defined herein (wherein $R^6$ is as hereinefore defined and Z represents a leaving atom or group or a cyano group), $R^6$ optionally being in protected form;

whereafter

(i) any protecting group present is removed to form a compound of the invention; and/or

(ii) where a compound of formula I as defined herein or a racemate thereof is obtained in the form of a base or acid and a salt is required, reacting said compound of formula I as defined herein or racemate thereof with respectively an acid or base to form a salt.

**2.** A process as claimed in claim 1 wherein $R^3$ represents phenyl substituted in the meta-position by:-

(i) a group of the formula as defined herein:-

-Y-W-M     II

in which Y represents a single bond, -S-, -0- or -NR- (in which R represents hydrogen, methyl, formyl or acetyl), W represents a $C_{2-4}$alkylene chain which may if desired be branched and M represents t group $-NR^7R^8R^9$ (wherein $R^7$, $R^8$ and $R^9$, which may be the same or different, each represents a $C_{1-7}$alkyl or aralkyl group;

(ii) a group of the formula III as defined in claim 1 in which $Y^1$ represents -0- or -S-; W represents a $C_{2-4}$alkylene chain which may if desired be branched; and $R^{10}$, $R^{11}$ and $R^{12}$, which may be the same or different, each represents hydrogen or methyl or $R^{10}$ and $R^{11}$ together represent a $C_2$ or $C_3$ alkylene chain such that $R^{10}$ and $R^{11}$ together with the intervening radical of formula IV

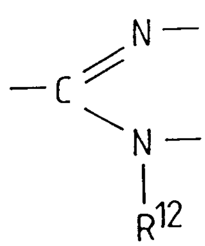

IV

form a 4,5-dihydroimidazol-2-yl or 3,4,5,6-tetrahydro- pyrimidin-2-yl ring, $R^{12}$ representing hydrogen or methyl; or $R^{11}$ and $R^{12}$ together represent a $C_4$ or $_5$ alkylene chain such that $R^{11}$ and $R^{12}$ together with the intervening nitrogen atom form a pyrrolidine or piperidine ring and $R^{10}$ represents hydrogen or methyl;

(iii) a group of the formula V

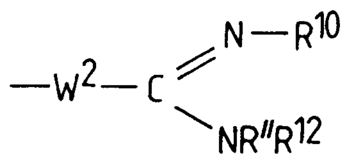

V

in which $W^1$ represents a $C_{2-4}$alkylene chain which may if desired be branched and $R^{10}$, $R^{11}$ and $R^{12}$ are as hereinbefore defined.

(iv) a group of the formula VI

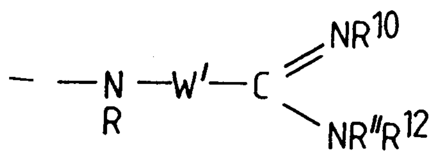

VI

in which $W^2$ represents a $C_{1-4}$ alkylene chain which may if desired be branched and $R^{10}$, $R^{11}$ and $R^{12}$ are as hereinbefore defined;

v) a group of the formula VII

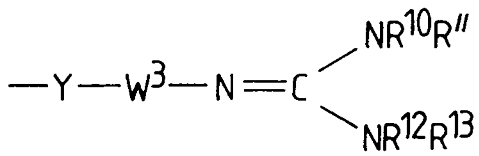

VII

in which Y is as hereinbefore defined, $W^3$ represents a $C_{2-4}$alkylene chain which may if desired be branched or where Y represents a single bond $W^3$ may represent a single bond or a $C_{1-4}$alkylene chain which may if desired be branched and $R^{10}$, $R^{11}$, $R^{12}$ and $R^{13}$, which may be the same or different, each represents hydrogen or methyl,

or $R^{10}$ and $R^{12}$ together represent a $C_2$ or $_3$ alkylene chain such that $R^{10}$ and $R^{12}$ together with the intervening radical of formula IVa

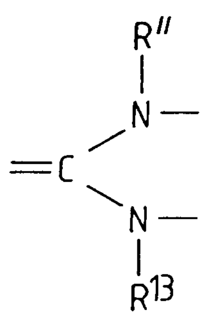

IVa

form a 4,5-dihydroimidazol-2-yl or 3,4,5,6-tetrahydropyrimidin-2-yl ring or tautomer thereof, $R^{11}$ and $R^{12}$ being independently selected from hydrogen or methyl,

or either $R^{10}$ and $R^{11}$ together or $R^{12}$ and $R^{13}$ together represent a $C_4$ or $_5$ alkylene chain such that either $R^{10}$ and $R^{11}$ together with the intervening nitrogen atom form a pyrrolidine or piperidine ring and $R^{12}$ and $R^{13}$ are independently selected from hydrogen and methyl or $R^{12}$ and $R^{13}$ together with the intervening nitrogen atom form a pyrrolidine or piperidine ring and $R^{10}$ and $R^{11}$ are independently selected from hydrogen and methyl; or

(vi) a group of the formula VIII in which Y and $W^2$ are as hereinbefore defined and A represents a carboxylic or sulphonic acid.

**3.** A process as claimed in claim 1 or 2 wherein the organic base, the organic acid or the quaternary ammonium salt is linked to phenyl at the meta position via oxygen or -NH.

**4.** A process as claimed in claim 1, 2 or 3 wherein $R^1$ represents chlorophenyl, dichlorophenyl, bromophenyl, fluorophenyl, difluorophenyl, trifluoromethylphenyl, nitrophenyl or cyanophenyl.

**5.** A process as claimed in any one of the preceding claims wherein X stands for $-CH_2-$, $-OCH_2-$, $SCH_2-$ or $-CH_2CH_2-$.

**6.** A process as claimed in any one of the preceding claims wherein $R^3$ stands for phenyl substituted in the meta-position by:

i) a group of formula II as defined in claim 1 in which W is $C_2$ or $C_3$ alkylene;

ii) a group of formula III as defined in claim 1 in which W is $CH_2CH_2$;

iii) a group of formula V as defined in claim 1 in which $W^1$ is a single bond or $-CH_2CH_2-$;

iv) a group of formula VI as defined in claim 1 in which $W^2$ is $-CH_2-$ or $-CH_2CH_2-$;

v) a group of formula VII as defined in claim 1 in which Y represents -0-, -S- or -NR- (wherein R is as hereinbefore defined) and $W^3$ is $-CH_2CH_2-$; or a group of formula VII in which Y represents a single bond and $W^3$ is a single bond - $CH_2-$ or $CH_2CH_2-$; or

vi) A group of formula VIII as defined in claim 2 in which $W^2$ is $-CH_2-$.

**7.** A process as claimed in any one of the preceeding claims wherein $R^3$ represents 3-(3-dimethylaminopropoxy)phenyl in the form of a quaternary ammonium salt, 3-(3-carboxymethoxy)phenyl, 3-guanidinophenyl; or a 3-trimethylammonium propoxy or 3-benzyldimethyl ammonimpropoxy group stabilised by an appropriate anion.

**8.** A process as claimed in any one of the preceding claims wherein $R^4$ and $R^5$ together with the intervening nitrogen atom represents a pyrrolidino, dimethylamino, diethylamino, N-allyl-N-methylamino, N- isopropyl-N-methylamino, $\Delta^3$-pyrrolino, or piperidino group.

**9.** A process as claimed in claim 1 which comprises the preparation of 2(R,S)-N-[2-(N-methyl-3,4-dichlorophenylacetamido)-2-(3-carboxymethoxyphenyl)-ethyl]pyrrolidine, (R,S)-N-[2-(N-methyl-3,4-dichlorophenylacetamido)-2-(3-trimethylammoniumpropoxyphenyl)-ethyl]-pyrrolidine iodide, (R,S)-N-[2-(N-methyl-3,4-dichlorophenylacetamido)-2-(3-benzyldimethylammoniumpropoxy-phenyl)-ethyl]-pyrrolidine chloride; or

(R,S)-N-[2-(N-methyl-3,4-dichlorophenylacetamido)-2-(3-guanidinophenyl)-ethyl]pyrrolidine and the salts thereof.

**10.** A process as claimed in any one of the preceeding claims wherein the compound of formula I as defined in claim 1 is in the form of a pharmaceutically acceptable salt.

**Patentansprüche**
**Patentansprüche für folgende Vertragsstaaten : BE, CH, DE, LI, NL**

1. Verbindungen der Formel I,

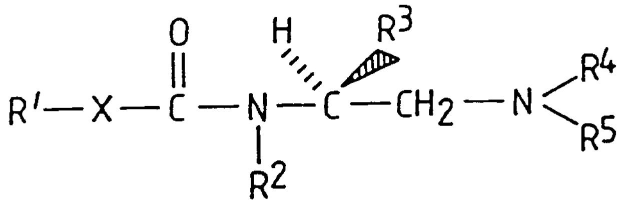

I

worin
$R^1$ für eine Halogenophenyl-, Dihalogenophenyl-, Nitrophenyl-, Cyanophenyl- oder Trifluoromethylphenyl-Gruppe steht;
X für eine einfache Bindung, $-CH_2$, $-OCH_2-$, $-SCH_2-$, $-S(O)-CH_2-$, $-S(O)_2-CH_2-$ oder $-CH_2-CH_2-$ steht;
$R^2$ für Wasserstoff oder $C_{1-3}$-Alkyl steht;
$R^3$ für eine Phenyl-Gruppe steht, die in der meta-Stellung durch ein Radikal, das eine organische Base, eine organische Säure oder ein quaternäres Ammoniumsalz enthält, substituiert ist und gegebenenfalls weiter substituiert ist durch Halogen, $C_{1-3}$-Alkyl, $C_{1-3}$-Alkoxy, Cyano, Nitro, Hydroxy, Amino, Mono- oder Di-($C_{1\ \text{oder}\ 2}$-alkyl)amino, Carboxamido, Mono- oder Di-($C_{1\ \text{oder}\ 2}$-alkyl)carboxamido, Ureido oder $C_{1-3}$-Acylamino;
$R^4$ und $R^5$, welche gleich oder verschieden sein können, jeweils für eine $C_{3-5}$-Alkenyl-, $C_{3-5}$-Alkinyl-, $C_{1-6}$-Alkyl- oder $C_{4-7}$-Cycloalkylalkyl-Gruppe stehen;
oder
$R^4$ und $R^5$ zusammen mit dem dazwischenliegenden Stickstoffatom einen 4- bis 7gliedrigen heterocyclischen Ring bilden, der gegebenenfalls ein weiteres aus Sauerstoff und Schwefel ausgewähltes Heteroatom enthält;
sowie Racemate davon und die Salze dieser Verbindungen oder Racemate;
wobei die Gruppe $-R^3$ so ausgewählt ist, daß die hier definierten Verbindungen der Formel I oder Racemate davon oder Salze der hier definierten Verbindungen der Formel I oder Racemate davon bei pH 7,4 einen log D von weniger als 0,5 aufweisen (wobei log D der Logarithmus des Verteilungskoeffizienten zwischen Octan-1-ol und wäßrigem Puffer ist).

2. Verbindungen nach Anspruch 1, worin $R^3$ für Phenyl steht, das in der meta-Stellung substituiert ist durch
(i) eine Gruppe der Formel II,

-Y-W-M II

worin Y für eine einfache Bindung, -S-, -O- oder -NR- (wobei R Wasserstoff, Methyl, Formyl oder Acetyl bedeutet) steht, W für eine gegebenenfalls verzweigte $C_{2-4}$-Alkylen-Kette steht und M für die Gruppe $-NR^7R^8R^9$ (wobei $R^7$, $R^8$ und $R^9$, welche gleich oder verschieden sein können, jeweils eine $C_{1-7}$-Alkyl- oder Aralkyl-Gruppe bedeuten) steht;
(ii) eine Gruppe der Formel III,

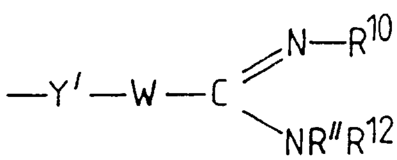

III

worin $Y^1$ für -O- oder -S- steht, W für eine gegebenenfalls verzweigte $C_{2-4}$-Alkylen-Kette steht und $R^{10}$, $R^{11}$ und $R^{12}$, welche gleich oder verschieden sein können, jeweils für Wasserstoff oder Methyl stehen oder $R^{10}$ und $R^{11}$ zusammen für eine $C_{2\ \text{oder}\ 3}$-Alkylen-Kette stehen, derart, daß $R^{10}$ und $R^{11}$ zusammen mit dem dazwischenliegenden Radikal der Formel IV

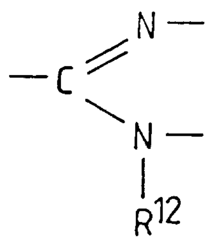

IV

einen 4,5-Dihydroimidazol-2-yl- oder 3,4,5,6-Tetrahydropyrimidin-2-yl-Ring bilden und $R^{12}$ für Wasserstoff oder Methyl steht oder $R^{11}$ und $R^{12}$ zusammen für eine $C_{4\ \text{oder}\ 5}$-Alkylen-Kette stehen, derart, daß $R^{11}$ und $R^{12}$ zusammen mit dem dazwischenliegenden Stickstoffatom einen Pyrrolidin- oder Piperidin-Ring bilden und $R^{10}$ für Wasserstoff oder Methyl steht;

(iii) eine Gruppe der Formel V,

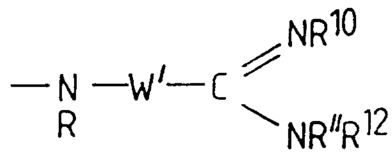

V

worin $W^1$ für eine gegebenenfalls verzweigte $C_{2-4}$-Alkylen-Kette steht und $R^{10}$, $R^{11}$ und $R^{12}$ die oben angegebenen Bedeutungen besitzen;

(iv) eine Gruppe der Formel VI,

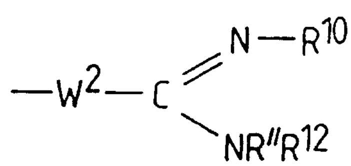

VI

worin $R^2$ für eine gegebenenfalls verzweigte $C_{1-4}$-Alkylen-Kette steht und $R^{10}$, $R^{11}$ und $R^{12}$ die oben angegebenen Bedeutungen besitzen;

(v) eine Gruppe der Formel VII,

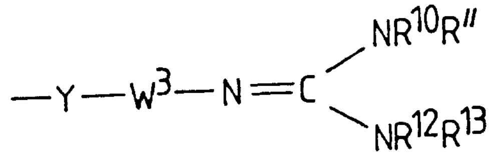

VII

worin Y die oben angegebene Bedeutung besitzt, $W^3$ für eine gegebenenfalls verzweigte $C_{2-4}$-Alkylen-Kette steht oder, wenn Y für eine einfache Bindung steht, $W^3$ auch für eine einfache Bindung

oder eine gegebenenfalls verzweigte $C_{1-4}$-Alkylen-Kette stehen kann und $R^{10}$, $R^{11}$, $R^{12}$ und $R^{13}$, welche gleich oder verschieden sein können, jeweils für Wasserstoff oder Methyl stehen oder $R^{10}$ und $R^{12}$ zusammen für eine $C_2$ oder $_3$-Alkylen-Kette stehen, derart, daß $R^{10}$ und $R^{12}$ zusammen mit dem dazwischenliegenden Radikal der Formel IVa

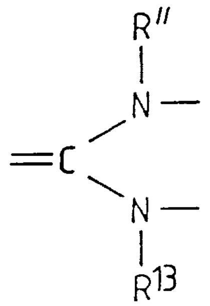

IVa

einen 4,5-Dihydroimidazol-2-yl- oder 3,4,5,6-Tetrahydropyrimidin-2-yl-Ring oder ein Tautomer davon bilden und $R^{11}$ und $R^{13}$ unabhängig für Wasserstoff oder Methyl stehen oder entweder $R^{10}$ und $R^{11}$ zusammen oder $R^{12}$ und $R^{13}$ zusammen für eine $C_{4\ \text{oder}\ 5}$-Alkylen-Kette stehen, derart, daß entweder $R^{10}$ und $R^{11}$ zusammen mit dem dazwischenliegenden Stickstoffatom einen Pyrrolidin- oder Piperidin-Ring bilden und $R^{12}$ und $R^{13}$ unabhängig für Wasserstoff oder Methyl stehen oder $R^{12}$ und $R^{13}$ zusammen mit dem dazwischenliegenden Stickstoffatom für einen Pyrrolidin- oder Piperidin-Ring stehen und $R^{10}$ und $R^{11}$ unabhängig für Wasserstoff oder Methyl stehen; oder
(vi) eine Gruppe der Formel VIII,

$-Y-W^2-A$ VIII

worin Y und $W^2$ die oben angegebenen Bedeutungen besitzen und A für eine Carbon- oder Sulfonsäure steht.

**3.** Verbindungen nach Anspruch 1 oder 2, bei welchen die organische Base, die organische Säure oder das quaternäre Anmoniumsalz über Sauerstoff oder -NH- an die meta-Stellung des Phenyls gebunden ist.

**4.** Verbindungen nach einem der vorhergehenden Ansprüche, worin $R^4$ und $R^5$, welche gleich oder verschieden sein können, jeweils für eine $C_{1-6}$-Alkyl-Gruppe oder eine $C_{3-5}$-Alkenyl-Gruppe stehen oder $R^4$ und $R^5$ zusammen mit dem dazwischenliegenden Stickstoffatom für einen 4- bis 7gliedrigen Ring stehen, der gegebenenfalls ein weiteres Heteroatom enthält.

**5.** Verbindungen nach einem der vorhergehenden Ansprüche, worin $R^1$ für Chlorophenyl, Dichlorophenyl, Bromophenyl, Fluorophenyl, Difluorophenyl, Trifluoromethylphenyl, Nitrophenyl oder Cyanophenyl steht.

**6.** Verbindungen nach einem der vorhergehenden Ansprüche, worin X für $-CH_2-$, $-OCH_2-$, $-SCH_2-$ oder $-CH_2CH_2-$ steht.

**7.** Verbindungen nach einem der vorhergehenden Ansprüche, worin $R^3$ für Phenyl steht, das in der meta-Stellung substituiert durch
(i) eine Gruppe der Formel II von Anspruch 2, worin W für $C_{2\ \text{oder}\ 3}$-Alkylen steht;
(ii) eine Gruppe der Formel III von Anspruch 2, worin W für $-CH_2CH_2-$ steht;
(iii) eine Gruppe der Formel V von Anspruch 2, worin $W^1$ für eine einfache Bindung oder $-CH_2CH_2-$ steht;
(iv) eine Gruppe der Formel VI von Anspruch 2, worin $W^2$ für $-CH_2-$ oder $-CH_2CH_2-$ steht;
(v) eine Gruppe der Formel VII von Anspruch 2, worin Y für -O-, -S- oder -NR- (wobei R die oben angegebene Bedeutung besitzt) steht und $W^3$ für $-CH_2CH_2-$ steht; oder eine Gruppe der Formel VII von Anspruch 2, worin Y für eine einfache Bindung steht und $W^3$ für eine einfache Bindung, $-CH_2-$

oder $-CH_2CH_2-$ steht; oder
(vi) eine Gruppe der Formel VIII von Anspruch 2, worin $W^2$ für $-CH_2-$ steht.

**8.** Verbindungen nach Anspruch 7, worin, wenn die Gruppe >N-R vorliegt, wie in Formel II, VII oder VIII von Anspruch 2, wenn Y >N-R bedeutet, oder wie in Formel V von Anspruch 2, R für Wasserstoff steht.

**9.** Verbindungen nach Anspruch 7 oder 8, worin, wenn die Gruppierungen $R^7$, $R^8$ oder $R^9$ anwesend sind, diese Gruppierungen, welche gleich oder verschieden sein können, aus Methyl oder Benzyl ausgewählt sind.

**10.** Verbindungen nach einem der vorhergehenden Ansprüche, worin $R^3$ für 3-(3-Dimethylaminopropoxy)-phenyl in Form eines quaternären Anmoniumsalzes, 3-(3-Carboxymethoxy)phenyl oder 3-Guanidinophenyl oder für eine durch ein geeignetes Anion stabilisierte 3-Trimethylammonium-propoxy- oder 3-Benzyldimethylammonium-propoxy-Gruppe steht.

**11.** Verbindungen nach einem der vorhergehenden Ansprüche, worin $R^4$ und $R^5$ zusammen mit dem dazwischenliegenden Stickstoffatom für eine Pyrrolidino-, Dimethylamino-, Diethylamino-, N-Allyl-N-methylamino-, N-Isopropyl-N-methylamio-, $\Delta^3$-Pyrrolidino- oder Piperidino-Gruppe stehen.

**12.** Verbindungen nach Anspruch 1, nämlich
2(R,S)-N-[2-(N-Methyl-3,4-dichlorophenylacetamido)-2-(3-carboxymethoxyphenyl)ethyl]pyrrolidin,
(R,S)-N-[2-(N-Methyl-3,4-dichlorophenylacetamido)-2-(3-trimethylammoniumpropoxyphenyl)ethyl]-pyrrolidin-jodid,
(R,S)-N-[2-(N-Methyl-3,4-dichlorophenylacetamido)-2-(3-benzyldimethylammoniumpropoxyphenyl)ethyl]-pyrrolidin-chlorid,
(R,S)-N-[2-(N-Methyl-3,4-dichlorophenylacetamido)-2-(3-guanidinophenyl)ethyl]pyrrolidin
sowie die Salze davon.

**13.** Verfahren zur Herstellung einer Verbindung der Formel I von Anspruch 1 oder eines Salzes davon, bei welchem
(a) zur Herstellung einer Verbindung der Formel I von Anspruch 1 eine Verbindung der Formel IX

$R^1$-X-$Y^2$ IX

worin $R^1$ und X die oben angegebenen Bedeutungen besitzen und $Y^2$ für eine Säure oder ein aktiviertes Derivat davon steht, mit einer Verbindung der Formel X,

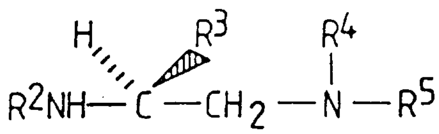

X

worin $R^2$, $R^3$, $R^4$ und $R^5$ die oben angegebenen Bedeutungen besitzen, oder einem Racemat davon, gegebenenfalls in geschützter Form, umgesetzt wird und nötigenfalls die so erhaltene Verbindung in die ungeschützte Form überführt wird, um eine Verbindung der Formel I von Anspruch 1 oder ein Racemat davon herzustellen, und gegebenenfalls diese Verbindung oder dieses Racemat unter Bildung eines Salzes mit eine Säure umgesetzt wird;
(b) zur Herstellung einer Verbindung der Formel I von Anspruch 1, worin $R^3$ für eine Phenyl-Gruppe steht, die in der meta-Stellung durch Hydroxy oder Amino und gegebenenfalls weiter wie oben definiert substituiert ist, eine entsprechende Verbindung, worin die genannte Phenyl-Gruppe in der meta-Stellung durch eine Acyloxy- oder Acylamino-Gruppe substituiert ist, hydrolysiert wird oder eine entsprechende Verbindung, worin die genannte Phenyl-Gruppe in der meta-Stellung durch eine Alkoxy- oder Benzyl-Gruppe substituiert ist, in die ungeschützte Form überführt wird;
(c) zur Herstellung einer Verbindung der Formel I von Anspruch 1, worin $R^3$ für eine Phenyl-Gruppe steht, die in der meta-Stellung durch Hydroxy oder Amino und gegebenenfalls weiter wie oben

definiert substituiert ist, und X für eine Gruppierung $-SO_2CH_2-$ steht, eine entsprechende Verbindung der Formel II oder III von Anspruch 2, worin X für eine Gruppierung $-SCH_2-$ oder $-SOCH_2-$steht, oxidiert wird;

(d) zur Herstellung einer Verbindung der Formel I von Anspruch 1, worin $R^3$ für eine Phenyl-Gruppe steht, die in der meta-Stellung durch Hydroxy oder Amino und gegebenenfalls weiter wie oben definiert substituiert ist, und X für eine Gruppierung $-SOCH_2-$ steht, eine entsprechende Verbindung der Formel II oder III von Anspruch 2, worin X für eine Gruppierung $-SCH_2-$, steht oxidiert wird;

(e) zur Herstellung einer Verbindung der Formel I von Anspruch 1, worin $R^3$ die oben angegebene Bedeutung besitzt, mit der Maßgabe, daß das eine organische Base, eine organische Säure oder ein quaternäres Ammoniumsalz enthaltende Radikal über Sauerstoff oder Schwefel an das Phenyl gebunden ist, eine Verbindung, die einer Verbindung der Formel I von Anspruch 1 entspricht, worin aber $R^3$ für eine meta-Hydroxyphenyl oder -Mercaptophenyl-Gruppe steht, die gegebenenfalls weiter wie oben substituiert ist, oder ein Racemat davon mit einer Verbindung der Formel $R^6Z$, worin Z für ein Austrittsatom oder eine Austrittsgruppe steht und $R^6$ für ein Radikal, das eine organische Base, eine organische Säure oder ein quaternäres Ammoniumsalz enthält, steht, wobei $R^6$ gegebenenfalls in geschützter Form vorliegt, umgesetzt wird, worauf (i) eine gegebenenfalls vorliegende Schutzgruppe unter Bildung einer erfindungsgemäßen Verbindung entfernt wird und/oder (ii), wenn eine Verbindung der Formel I von Anspruch 1 oder ein Racemat davon in Form einer Base oder einer Säure erhalten wird und ein Salz gewünscht wird, diese Verbindung der Formel I von Anspruch 1 oder das Racemat davon zur Bildung eines Salzes mit einer Säure bzw. einer Base umgesetzt wird;

(f) zur Herstellung einer Verbindung der Formel I von Anspruch 1, worin $R^3$ die oben angegebene Bedeutung besitzt, mit der Maßgabe, daß der Substituent in der meta-Stellung ein quaternäres Ammoniumsalz enthält und über Sauerstoff, -NH-oder Schwefel an das Phenyl gebunden ist, eine Verbindung, welche einer Verbindung der Formel I von Anspruch 1 entspricht, worin aber $R^3$ für eine ein tertiäres Amin enthaltende Gruppe steht, quaternisiert wird;

(g) zur Herstellung einer Verbindung der Formel I von Anspruch 1, worin $R^3$ die oben angegebene Bedeutung besitzt, mit der Maßgabe, daß der Substituent in der meta-Stellung über -NH- an das Phenyl gebunden ist, eine Verbindung, die einer Verbindung der Formel I von Anspruch 1 entspricht, worin aber $R^3$ für eine meta-Aminophenyl-Gruppe steht (die gegebenenfalls weiter wie oben definiert substituiert ist), oder ein Racemat davon mit einer Verbindung der Formel $R^6Z$, worin $R^6$ die oben angegebene Bedeutung besitzt und Z für ein Austrittsatom oder eine Austrittsgruppe oder eine Cyano-Gruppe steht, wobei $R^6$ gegebenenfalls in geschützter Form vorliegt, umgesetzt wird, worauf (i) eine gegebenenfalls anwesende Schutzgruppe unter Bildung einer erfindungsgemäßen Verbindung entfernt wird und/oder (ii), wenn eine Verbindung der Formel I von Anspruch 1 oder ein Racemat davon in Form einer Base oder Säure erhalten wird und ein Salz gewünscht wird, diese Verbindung der Formel I von Anspruch 1 oder das Racemat davon zur Bildung eines Salzes mit einer Säure bzw. Base umgesetzt wird.

**14.** Pharmazeutische Zusammensetzung, welche als aktiven Bestandteil mindestens eine Verbindung der Formel I von Anspruch 1 oder ein Racemat davon oder ein pharmazeutisch zulässiges Salz dieser Verbindung oder dieses Racemats gemeinsam mit einem pharmazeutisch zulässigen Träger- oder Verdünnungsmittel enthält.

**Patentansprüche für folgenden Vertragsstaat : ES**

**1.** Verfahren zur Herstellung einer Verbindung der Formel I,

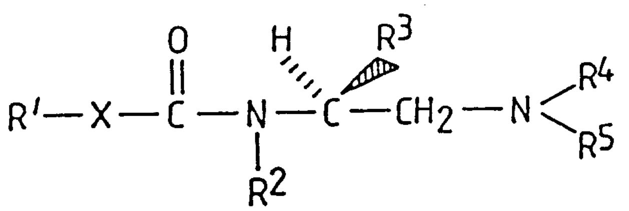

I

worin
$R^1$ für eine Halogenophenyl-, Dihalogenophenyl-, Nitrophenyl-, Cyanophenyl- oder Trifluoromethylphenyl-Gruppe steht;
X für eine einfache Bindung, $-CH_2$, $-OCH_2-$, $-SCH_2-$, $-S(O)-CH_2-$, $-S(O)_2-CH_2-$ oder $-CH_2-CH_2-$ steht;
$R^2$ für Wasserstoff oder $C_{1-3}$-Alkyl steht;
$R^3$ für eine Phenyl-Gruppe steht, die in der meta-Stellung durch ein Radikal, das eine organische Base, eine organische Säure oder ein quaternäres Ammoniumsalz enthält, substituiert ist und gegebenenfalls weiter substituiert ist durch Halogen, $C_{1-3}$-Alkyl, $C_{1-3}$-Alkoxy, Cyano, Nitro, Hydroxy, Amino, Mono- oder Di-($C_1$ oder $_2$-alkyl)amino, Carboxamido, Mono- oder Di-($C_1$ oder $_2$-alkyl)carboxamido, Ureido oder $C_{1-3}$-Acylamino;
$R^4$ und $R^5$, welche gleich oder verschieden sein können, jeweils für eine $C_{3-5}$-Alkenyl-, $C_{3-5}$-Alkinyl-, $C_{1-6}$-Alkyl- oder $C_{4-7}$-Cycloalkylalkyl-Gruppe stehen;
oder
$R^4$ und $R^5$ zusammen mit dem dazwischenliegenden Stickstoffatom einen 4- bis 7gliedrigen heterocyclischen Ring bilden, der gegebenenfalls ein weiteres aus Sauerstoff und Schwefel ausgewähltes Heteroatom enthält;
oder eines Racemats davon oder eines Salzes dieser Verbindung oder Racemats;
wobei die Gruppe $-R^3$ so ausgewählt ist, daß die hier definierten Verbindungen der Formel I oder Racemate davon oder Salze der hier definierten Verbindungen der Formel I oder Racemate davon bei pH 7,4 einen log D von weniger als 0,5 aufweisen (wobei log D der Logarithmus des Verteilungskoeffizienten zwischen Octan-1-ol und wäßrigem Puffer ist),
bei welchem,

(a) zur Herstellung einer oben definierten Verbindung der Formel I eine Verbindung der Formel IX

$R^1$-X-$Y^2$ IX

worin $R^1$ und X die oben angegebenen Bedeutungen besitzen und $Y^2$ für eine Säure oder ein aktiviertes Derivat davon steht, mit einer Verbindung der Formel X,

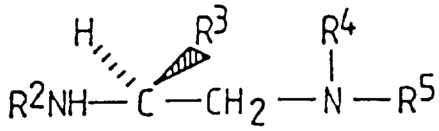

X

worin $R^2$, $R^3$, $R^4$ und $R^5$ die oben angegebenen Bedeutungen besitzen, oder einem Racemat davon, gegebenenfalls in geschützter Form, umgesetzt wird und nötigenfalls die so erhaltene Verbindung in die ungeschützte Form überführt wird, um eine Verbindung der obigen Formel I oder ein Racemat davon herzustellen, und gegebenenfalls diese Verbindung oder dieses Racemat unter Bildung eines Salzes mit eine Säure umgesetzt wird;

(b) zur Herstellung einer oben definierten Verbindung der Formel I, worin $R^3$ für eine Phenyl-Gruppe steht, die in der meta-Stellung durch Hydroxy oder Amino und gegebenenfalls weiter wie oben definiert substituiert ist, eine entsprechende Verbindung, worin die genannte Phenyl-Gruppe in der meta-Stellung durch eine Acyloxy- oder Acylamino-Gruppe substituiert ist, hydrolysiert wird oder eine entsprechende Verbindung, worin die genannte Phenyl-Gruppe in der meta-Stellung durch eine Alkoxy- oder Benzyl-Gruppe substituiert ist, in die ungeschützte Form überführt wird;

(c) zur Herstellung einer oben definierten Verbindung der Formel I, worin $R^3$ für eine Phenyl-Gruppe steht, die in der meta-Stellung durch Hydroxy oder Amino und gegebenenfallS weiter wie oben definiert substituiert ist, und X für eine Gruppierung $-SO_2CH_2-$ steht, eine entsprechende Verbindung der Formel II

-Y-W-M II

oder III

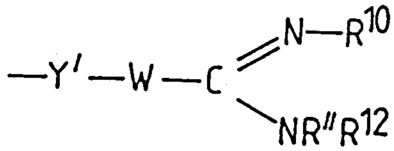

III

worin X für eine Gruppierung $-SCH_2-$ oder $-SOCH_2-$steht, oxidiert wird;

(d) zur Herstellung einer oben definierten Verbindung der Formel I, worin $R^3$ für eine Phenyl-Gruppe steht, die in der meta-Stellung durch Hydroxy oder Amino und gegebenenfalls weiter wie oben definiert substituiert ist, und X für eine Gruppierung $-SOCH_2-$ steht, eine entsprechende Verbindung der obigen Formel II oder III, worin X für eine Gruppierung $-SCH_2-$, steht oxidiert wird;

(e) zur Herstellung einer oben definierten Verbindung der Formel I, worin $R^3$ die oben angegebene Bedeutung besitzt, mit der Maßgabe, daß das eine organische Base, eine organische Säure oder ein quaternäres Ammoniumsalz enthaltende Radikal über Sauerstoff oder Schwefel an das Phenyl gebunden ist, eine Verbindung, die einer Verbindung der obigen Formel I entspricht, worin aber $R^3$ für eine meta-Hydroxyphenyl oder -Mercaptophenyl-Gruppe steht, die gegebenenfalls weiter wie oben substituiert ist, oder ein Racemat davon mit einer Verbindung der Formel $R^6Z$, worin Z für ein Austrittsatom oder eine Austrittsgruppe steht und $R^6$ für ein Radikal, das eine organische Base, eine organische Säure oder ein quaternäres Ammoniumsalz enthält, steht, wobei $R^6$ gegebenenfalls in geschützter Form vorliegt, umgesetzt wird, worauf (i) eine gegebenenfalls vorliegende Schutzgruppe unter Bildung einer erfindungsgemäßen Verbindung entfernt wird und/oder (ii), wenn eine Verbindung der obigen Formel I oder ein Racemat davon in Form einer Base oder einer Säure erhalten wird und ein Salz gewünscht wird, diese Verbindung der obigen Formel I oder das Racemat davon zur Bildung eines Salzes mit einer Säure bzw. einer Base umgesetzt wird;

(f) zur Herstellung einer oben definierten Verbindung der Formel I, worin $R^3$ die oben angegebene Bedeutung besitzt, mit der Maßgabe, daß der Substituent in der meta-Stellung ein quaternäres Ammoniumsalz enthält und über Sauerstoff, -NH-oder Schwefel an das Phenyl gebunden ist, eine Verbindung, welche einer Verbindung der obigen Formel I entspricht, worin aber $R^3$ für eine ein tertiäres Amin enthaltende Gruppe steht, quaternisiert wird;

(g) zur Herstellung einer oben definierten Verbindung der Formel I, worin $R^3$ die oben angegebene Bedeutung besitzt, mit der Maßgabe, daß der Substituent in der meta-Stellung über -NH- an das Phenyl gebunden ist, eine Verbindung, die einer Verbindung der obigen Formel I entspricht, worin aber $R^3$ für eine meta-Aminophenyl-Gruppe steht (die gegebenenfalls weiter wie oben definiert substituiert ist), oder ein Racemat davon mit einer Verbindung der Formel $R^6Z$, worin $R^6$ die oben angegebene Bedeutung besitzt und Z für ein Austrittsatom oder eine Austrittsgruppe oder eine Cyano-Gruppe steht, wobei $R^6$ gegebenenfalls in geschützter Form vorliegt, umgesetzt wird, worauf (i) eine gegebenenfalls anwesende Schutzgruppe unter Bildung einer erfindungsgemäßen Verbindung entfernt wird und/oder (ii), wenn eine Verbindung der obigen Formel I oder ein Racemat davon in Form einer Base oder Säure erhalten wird und ein Salz gewünscht wird, diese Verbindung der obigen Formel I oder das Racemat davon zur Bildung eines Salzes mit einer Säure bzw. Base umgesetzt wird.

**2.** Verfahren nach Anspruch 1, bei welchem $R^3$ für Phenyl steht, das in der meta-Stellung substituiert ist durch

(i) eine Gruppe der Formel II,

-Y-W-M II

worin Y für eine einfache Bindung, -S-, -O- oder -NR- (wobei R Wasserstoff, Methyl, Formyl oder Acetyl bedeutet) steht, W für eine gegebenenfalls verzweigte $C_{2-4}$-Alkylen-Kette steht und M für die Gruppe $-NR^7R^8R^9$ (wobei $R^7$, $R^8$ und $R^9$, welche gleich oder verschieden sein können, jeweils eine $C_{1-7}$-Alkyl- oder Aralkyl-Gruppe bedeuten) steht;

(ii) eine Gruppe der Formel III von Anspruch 1, worin $Y^1$ für -O- oder -S- steht, W für eine gegebenenfalls verzweigte $C_{2-4}$-Alkylen-Kette steht und $R^{10}$, $R^{11}$ und $R^{12}$, welche gleich oder verschieden sein können, jeweils für Wasserstoff oder Methyl stehen oder $R^{10}$ und $R^{11}$ zusammen

für eine $C_2$ oder $_3$-Alkylen-Kette stehen, derart, daß $R^{10}$ und $R^{11}$ zusammen mit dem dazwischenliegenden Radikal der Formel IV

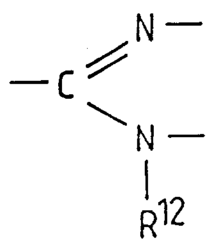

IV

einen 4,5-Dihydroimidazol-2-yl- oder 3,4,5,6-Tetrahydropyrimidin-2-yl-Ring bilden und $R^{12}$ für Wasserstoff oder Methyl steht oder $R^{11}$ und $R^{12}$ zusammen für eine $C_4$ oder $_5$-Alkylen-Kette stehen, derart, daß $R^{11}$ und $R^{12}$ zusammen mit dem dazwischenliegenden Stickstoffatom einen Pyrrolidin- oder Piperidin-Ring bilden und $R^{10}$ für Wasserstoff oder Methyl steht;
(iii) eine Gruppe der Formel V,

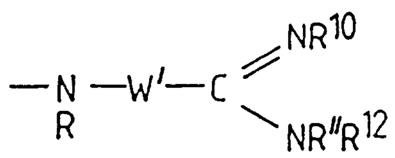

V

worin $W^1$ für eine gegebenenfalls verzweigte $C_{2-4}$-Alkylen-Kette steht und $R^{10}$, $R^{11}$ und $R^{12}$ die oben angegebenen Bedeutungen besitzen;
(iv) eine Gruppe der Formel VI,

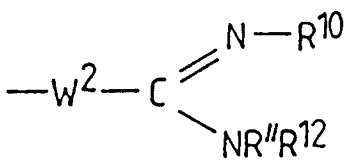

VI

worin $R^2$ für eine gegebenenfalls verzweigte $C_{1-4}$-Alkylen-Kette steht und $R^{10}$, $R^{11}$ und $R^{12}$ die oben angegebenen Bedeutungen besitzen;
(v) eine Gruppe der Formel VII,

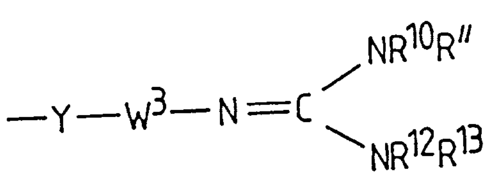

VII

worin Y die oben angegebene Bedeutung besitzt, $W^3$ für eine gegebenenfalls verzweigte $C_{2-4}$-Alkylen-Kette steht oder, wenn Y für eine einfache Bindung steht, $W^3$ auch für eine einfache Bindung oder eine gegebenenfalls verzweigte $C_{1-4}$-Alkylen-Kette stehen kann und $R^{10}$, $R^{11}$, $R^{12}$ und $R^{13}$, welche gleich oder verschieden sein können, jeweils für Wasserstoff oder Methyl stehen oder $R^{10}$

und $R^{12}$ zusammen für eine $C_2$ oder $_3$-Alkylen-Kette stehen, derart, daß $R^{10}$ und $R^{12}$ zusammen mit dem dazwischenliegenden Radikal der Formel IVa

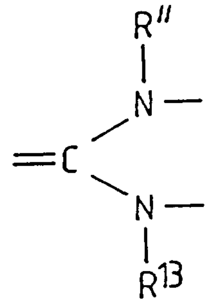

IVa

einen 4,5-Dihydroimidazol-2-yl- oder 3,4,5,6-Tetrahydropyrimidin-2-yl-Ring oder ein Tautomer davon bilden und $R^{11}$ und $R^{13}$ unabhängig für Wasserstoff oder Methyl stehen oder entweder $R^{10}$ und $R^{11}$ zusammen oder $R^{12}$ und $R^{13}$ zusammen für eine $C_4$ oder $_5$-Alkylen-Kette stehen, derart, daß entweder $R^{10}$ und $R^{11}$ zusammen mit dem dazwischenliegenden Stickstoffatom einen Pyrrolidin- oder Piperidin-Ring bilden und $R^{12}$ und $R^{13}$ unabhängig für Wasserstoff oder Methyl stehen oder $R^{12}$ und $R^{13}$ zusammen mit dem dazwischenliegenden Stickstoffatom für einen Pyrrolidin- oder Piperidin-Ring stehen und $R^{10}$ und $R^{11}$ unabhängig für Wasserstoff oder Methyl stehen; oder
(vi) eine Gruppe der Formel VIII,
worin Y und $W^2$ die oben angegebenen Bedeutungen besitzen und A für eine Carbon- oder Sulfonsäure steht.

**3.** Verfahren nach Anspruch 1 oder 2, bei welchem die organische Base, die organische Säure oder das quaternäre Ammoniumsalz über Sauerstoff oder -NH- an die meta-Stellung des Phenyls gebunden ist.

**4.** Verfahren nach Anspruch 1, 2 oder 3, bei welchem $R^1$ für Chlorophenyl, Dichlorophenyl, Bromophenyl, Fluorophenyl, Difluorophenyl, Trifluoromethylphenyl, Nitrophenyl oder Cyanophenyl steht.

**5.** Verfahren nach einem der vorhergehenden Ansprüche, bei welchem X für $-CH_2-$, $-OCH_2-$, $-SCH_2-$ oder $-CH_2CH_2-$steht.

**6.** Verfahren nach einem der vorhergehenden Ansprüche, bei welchem $R^3$ für Phenyl steht, das in der meta-Stellung substituiert durch
(i) eine Gruppe der Formel II von Anspruch 1, worin W für $C_2$ oder $_3$-Alkylen steht;
(ii) eine Gruppe der Formel III von Anspruch 1, worin W für $-CH_2CH_2-$ steht;
(iii) eine Gruppe der Formel V von Anspruch 1, worin $W^1$ für eine einfache Bindung oder $-CH_2CH_2-$ steht;
(iv) eine Gruppe der Formel VI von Anspruch 1, worin $W^2$ für $-CH_2-$ oder $-CH_2CH_2-$ steht;
(v) eine Gruppe der Formel VII von Anspruch 1, worin Y für -O-, -S- oder -NR- (wobei R die oben angegebene Bedeutung besitzt) steht und $W^3$ für $-CH_2CH_2-$ steht; oder eine Gruppe der Formel VII, worin Y für eine einfache Bindung steht und $W^3$ für eine einfache Bindung, $-CH_2-$ oder $-CH_2CH_2-$ steht; oder
(vi) eine Gruppe der Formel VIII von Anspruch 2, worin $W^2$ für $-CH_2-$ steht.

**7.** Verfahren nach einem der vorhergehenden Ansprüche, bei welchem $R^3$ für 3-(3-Dimethylaminopropoxy)phenyl in Form eines quaternären Ammoniumsalzes, 3-(3-Carboxymethoxy)phenyl oder 3-Guanidinophenyl oder für eine durch ein geeignetes Anion stabilisierte 3-Trimethylammonium-propoxy- oder 3-Benzyldimethylammonium-propoxy-Gruppe steht.

**8.** Verfahren nach einem der vorhergehenden Ansprüche, bei welchem $R^4$ und $R^5$ zusammen mit dem dazwischenliegenden Stickstoffatom für eine Pyrrolidino-, Dimethylamino-, Diethylamino-, N-Allyl-N-methylamino-, N-Isopropyl-N-methylamio-, $\Delta^3$-Pyrrolidino- oder Piperidino-Gruppe stehen.

9. Verfahren nach Anspruch 1, bei welchem die folgenden Verbindungen hergestellt werden:
2(R,S)-N-[2-(N-Methyl-3,4-dichlorophenylacetamido)-2-(3-carboxymethoxyphenyl)ethyl]pyrrolidin,
(R,S)-N-[2-(N-Methyl-3,4-dichlorophenylacetamido)-2-(3-trimethylammoniumpropoxyphenyl)ethyl]-pyrrolidin-jodid,
(R,S)-N-[2-(N-Methyl-3,4-dichlorophenylacetamido)-2-(3-benzyldimethylammoniumpropoxyphenyl)ethyl]-pyrrolidin-chlorid,
(R,S)-N-[2-(N-Methyl-3,4-dichlorophenylacetamido)-2-(3-guanidinophenyl)ethyl]pyrrolidin
sowie die Salze davon.

10. Verfahren nach einem der vorhergehenden Ansprüche, bei welchem die Verbindung der Formel I von Anspruch 1 in Form eines pharmazeutisch zulässigen Salzes erhalten wird.

**Revendications**
**Revendications pour les Etats contractants suivants : BE, CH, DE, LI, NL**

1. Composé de formule (I) :

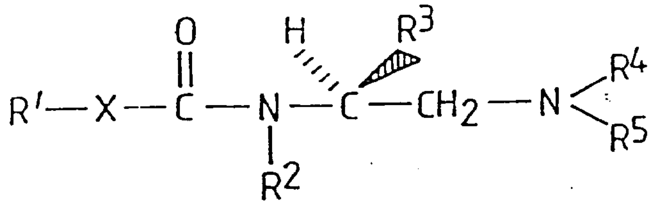

(I)

[où $R^1$ représente un radical halophényle, dihalophényle, nitrophényle, cyanophényle ou trifluorométhylphényle;

X représente une simple liaison, $-CH_2-$, $-OCH_2-$, $-SCH_2-$, $-S(O)-CH_2-$, $-S(O)_2-CH_2-$ ou $-CH_2-CH_2-$;

$R^2$ représente un hydrogène ou alcoyle en $C_{1-3}$;

$R^3$ représente un radical phényle substitué en position méta par un radical contenant une base organique, un acide organique ou un sel d'ammonium quaternaire et, facultativement, substitué par ailleurs par halogène, $C^{1-3}$ alcoyle, $C_{1-3}$ alcoxy, cyano, nitro, hydroxy, amino, mono- ou di-($C_{1\ ou\ 2}$ alcoyl)amino, carboxamido, mono- ou di-($C_{1\ ou\ 2}$ alcoyl)carboxamido, uréido ou $C_{1-3}$ acylamino;

$R^4$ et $R^5$, qui peuvent être les mêmes ou différents, représentent chacun un radical $C_{3-5}$ alcényle, $c_{3-5}$ alcynyle, $C_{1-6}$ alcoyle ou $C_{4-7}$ cycloalcoyle; ou

$R^4$ et $R^5$ représentent ensemble, avec l'atome d'azote intermédiaire, un hétérocycle à 4-7 membres, qui contient facultativement un hétéroatome supplémentaire choisi parmi oxygène et soufre] ou le racémique de celui-ci, et les sels de ce composé ou racémique; le groupe $R^3$ étant choisi de manière à ce que le composé de formule (I) ou son racémique ou le sel de ce composé de formule (I) ou de son racémique ait un log D inférieur à 0,5 à pH 7,4 (où log D est le logarithme du coefficient de distribution entre le 1-octanol et un tampon aqueux).

2. Composé suivant la revendication 1, dans lequel $R^3$ représente phényle substitué en position méta par :
(i) un groupe de formule :

-Y-W-M (II)

dans laquelle Y représente une liaison simple, -S-, -O- ou -NR- (où R représente hydrogène, méthyle, formyle ou acétyle), W représente une chaîne alcoylène en $C_{2-4}$, qui peut être branchée si désiré, et M représente le groupe $-NR^7R^8R^9$ (où $R^7$, $R^8$ et $R^9$, qui peuvent être les mêmes ou différents, représentent chacun un groupe alcoyle ou aralcoyle en $C_{1-7}$);

(ii) un groupe de formule (III) :

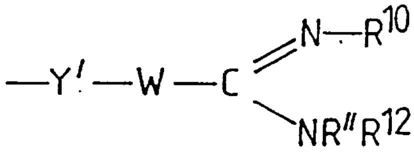

(III)

dans lequel $Y^1$ représente -O- ou -S-; W représente une chaîne alcoylène en $C_{2-4}$, qui peut être branchée si désiré, et $R^{10}$, $R^{11}$ et $R^{12}$, qui peuvent être les mêmes ou différents, représentent chacun hydrogène ou méthyle ou $R^{10}$ et $R^{11}$ représentent ensemble une chaîne alcoylène en $C_2$ ou en $C_3$, de manière que $R^{10}$ et $R^{11}$ forment, avec le radical intermédiaire de formule (IV) :

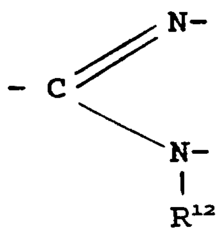

(IV)

un cycle 4,5-dihydroimidazol-2-yle ou 3,4,5,6-tétrahydro-pyrimidin-2-yle, $R^{12}$ représentant hydrogène ou méthyle; ou $R^{11}$ et $R^{12}$ représentent ensemble une chaîne alcoylène en $C_4$ ou en $C_5$, de manière que $R^{11}$ et $R^{12}$ forment avec l'atome d'azote intermédiaire un cycle pyrrolidine ou pipéridine et $R^{10}$ représente hydrogène ou méthyle;

(iii) un groupe de formule (V) :

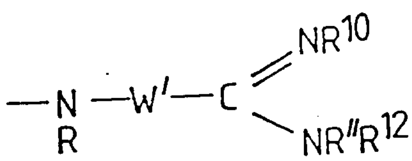

(V)

dans lequel $W^1$ représente une chaîne alcoylène en $C_{2-4}$, qui peut être branchée si désiré, et $R^{10}$, $R^{11}$ et $R^{12}$ sont tels que définis ci-dessus;

(iv) un groupe de formule (VI) :

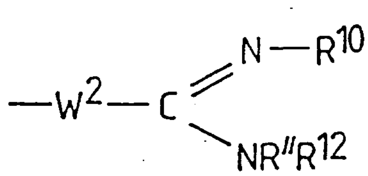

(VI)

dans lequel $W^2$ représente une chaîne alcoylène en $C_{1-4}$, qui peut être branchée si désiré, et $R^{10}$, $R^{11}$ et $R^{12}$ sont tels que définis ci-dessus;

(v) un groupe de formule (VII) :

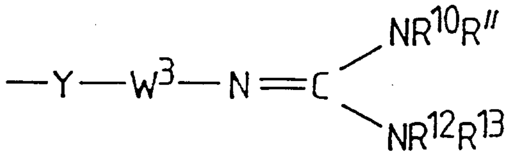

(VII)

dans lequel Y est tel que défini ci-dessus, $W^3$ représente une chaîne alcoylène en $C_{2-4}$, qui peut être branchée si désiré, ou, quand Y représente une liaison simple, $W^3$ peut représenter une liaison simple ou une chaîne alcoylène en $C_{1-4}$, qui peut être branchée si désiré, et $R^{10}$, $R^{11}$, $R^{12}$ et $R^{13}$, qui peuvent être les mêmes ou différents, représentent chacun hydrogène ou méthyle,

ou $R^{10}$ et $R^{12}$ représentent ensemble une chaîne alcoylène en $C_2$ ou en $C_3$, de manière que $R^{10}$ et $R^{12}$ forment, avec le radical intermédiaire de formule (IVa) :

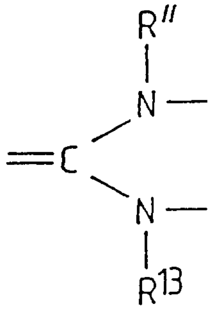

(IVa)

un cycle 4,5-dihydroimidazol-2-yle ou 3,4,5,6-tétrahydro-pyrimidin-2-yle, ou un tautomère correspondant, $R^{11}$ et $R^{12}$ étant choisis indépendamment parmi hydrogène et méthyle,

ou $R^{10}$ et $R^{11}$ ensemble, ou $R^{12}$ et $R^{13}$ ensemble représentent une chaîne alcoylène en $C_4$ ou en $C_5$, de manière que soit $R^{10}$ et $R^{11}$ forment avec l'atome d'azote intermédiaire un cycle pyrrolidine ou pipéridine et, $R^{12}$ et $R^{13}$ sont choisis indépendamment parmi hydrogène et méthyle, soit $R^{12}$ et $R^{13}$ forment avec l'atome d'azote intermédiaire un cycle pyrrolidine ou pipéridine et, $R^{10}$ et $R^{11}$ sont choisis indépendamment parmi hydrogène et méthyle; ou

(vi) un groupe de formule (VIII) :

$-Y-W^2-A$ (VIII)

dans lequel Y et $W^2$ sont tels que définis précédemment, et A représente un acide carboxylique ou sulfonique.

**3.** Composé suivant la revendication 1 ou 2, dans lequel la base organique, l'acide organique ou le sel d'ammonium quaternaire est lié au phényle en position méta via un oxygène ou un -NH-.

**4.** Composé suivant l'une quelconque des revendications précédentes, dans lequel $R^4$ et $R^5$, qui peuvent être les mêmes ou différents, sont chacun un radical alcoyle en $C_{1-6}$ ou un radical alcényle en $C_{3-5}$ ou $R^4$ et $R^5$, ensemble, représentent avec l'atome d'azote intermédiaire un cycle à 4-7 membres, contenant facultativement un hétéroatome supplémentaire.

**5.** Composé suivant l'une quelconque des revendications précédentes, dans lequel $R^1$ représente chlorophényle, dichlorophényle, bromophényle, fluorophényle, difluorophényle, trifluorométhylphényle, nitrophényle ou cyanophényle.

**6.** Composé suivant l'une quelconque des revendications précédentes, dans lequel X représente $-CH_2-$, $-OCH_2-$, $-SCH_2-$ ou $-CH_2CH_2-$.

**7.** Composé suivant l'une quelconque des revendications précédentes, dans lequel $R^3$ représente phényle substitué en position méta par :

i) un groupe de formule (II) tel que défini dans la revendication 2, dans lequel W est alcoylène en $C_2$ ou en $C_3$;

ii) un groupe de formule (III) tel que défini dans la revendication 2, dans lequel W est $-CH_2CH_2-$;

iii) un groupe de formule (V) tel que défini dans la revendication 2, dans lequel $W^1$ est une liaison simple ou $-CH_2CH_2-$;

iv) un groupe de formule (VI) tel que défini dans la revendication 2, dans lequel $W^2$ est $-CH_2-$ ou $-CH_2CH_2-$;

v) un groupe de formule (VII) tel que défini dans la revendication 2, dans lequel Y représente -O-, -S-ou -NR-

(où R est tel que défini ci-dessus) et $W^3$ est $-CH_2CH_2-$; ou un groupe de formule (VII) tel que défini dans la revendication 2, dans lequel Y représente une liaison simple et $W^3$ est une liaison simple, $-CH_2-$ ou $-CH_2CH_2-$; ou

vi) un groupe de formule (VIII) tel que défini dans la revendication 2, dans lequel $W^2$ est $-CH_2-$.

**8.** Composé suivant la revendication 7, dans lequel quand le radical >N-R est présent, comme dans les formules (II), (VII) ou (VIII) telles que définies dans la revendication 2, dans lesquelles Y représente >N-R, ou dans la formule (V) telle que définie dans la revendication 2, R est hydrogène.

**9.** Composé suivant la revendication 7 ou 8, dans lequel quand les radicaux $R^7$, $R^8$ ou $R^9$ sont présents, ces radicaux, qui peuvent être les mêmes ou différents, sont choisis parmi méthyle et benzyle.

**10.** Composé suivant l'une quelconque des revendications précédentes, dans lequel $R^3$ représente 3-(3-diméthylaminopropoxy)phényle, sous la forme d'un sel d'ammonium quaternaire, 3-(3-carboxyméthoxy)phényle, 3-guanidinophényle; ou un radical 3-triméthylammoniumpropoxy ou 3-benzyldiméthylammoniumpropoxy stabilisé par un anion approprié.

**11.** Composé suivant l'une quelconque des revendications précédentes, dans lequel $R^4$ et $R^5$, ensemble avec l'atome d'azote intermédiaire, représentent un radical pyrrolidino, diméthylamino, diéthylamino, N-allyl-N-méthylamino, N-isopropyl-N-méthylamino, $\Delta^3$-pyrrolino ou pipéridino.

**12.** Composé suivant la revendication 1, qui est la 2(R,S)-N-[2-(N-méthyl-3,4-dichlorophénylacétamido)-2-(3-carboxyméthoxyphényl)-éthyl]pyrrolidine, l'iodure de (R,S)-N-[2-(N-méthyl-3,4-dichlorophénylacétamido)-2-(3-triméthylammonium-propoxyphényl)-éthyl]pyrrolidine, le chlorure de (R,S)-N-[2-(N-méthyl-3,4-dichlorophénylacétamido)-2-(3-benzyldiméthylammonium-propoxyphényl)-éthyl]pyrrolidine; la (R,S)-N-[2-(N-méthyl-3,4-dichlorophénylacétamido)-2-(3-guanidinophényl)-éthyl]pyrrolidine et les sels de ceux-ci.

**13.** Procédé pour préparer un composé de formule (I) tel que défini dans la revendication 1 ou un sel de celui-ci, qui comprend :

a) pour la préparation d'un composé de formule (I) tel que défini dans la revendication 1, la réaction d'un composé de formule (IX)

$R^1$-X-$Y^2$ (IX)

dans lequel $R^1$ et X sont tels que définis ci-dessus et $Y^2$ représente un acide ou un dérivé activé de celui-ci, avec un composé de formule (X)

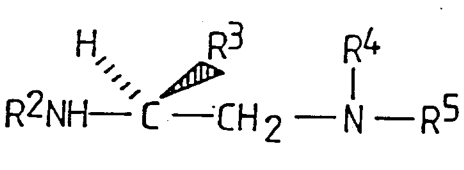

(X)

(où $R^2$, $R^3$, $R^4$ et $R^5$ sont tels que définis ci-dessus), ou d'un racémique de celui-ci, facultativement

sous une forme protégée, et, quand c'est nécessaire, la déprotection du composé ainsi obtenu pour former un composé de formule (I) tel que défini dans la revendication 1 ou un racémique de celui-ci, et, si désiré, la réaction de ce composé ou racémique avec un acide pour former un sel;

b) pour la préparation d'un composé de formule (I) tel que défini dans la revendication 1, dans lequel $R^3$ représente un radical phényle substitué en méta par hydroxy ou amino et facultativement substitué par ailleurs, comme défini ci-dessus, l'hydrolyse d'un composé correspondant, dans lequel ce radical phényle est substitué en méta par un radical acyloxy ou acylamino, ou par la déprotection d'un composé correspondant dans lequel ce radical phényle est substitué en méta par un radical alcoyloxy ou benzyle;

c) pour la préparation d'un composé de formule (I) tel que défini dans la revendication 1, dans lequel $R^3$ représente un radical phényle substitué en méta par hydroxy ou amino et facultativement substitué par ailleurs comme défini ci-dessus, dans lequel X représente un radical $-SOCH_2-$, l'oxydation d'un composé correspondant de formule (II) ou (III) tel que défini dans la revendication 2, dans lequel X représente un radical $-SCH_2-$ou $-SOCH_2-$;

d) pour la préparation de composés de formule (I) tel que défini dans la revendication 1, dans lequel $R^3$ représente un radical phényle substitué en méta par hydroxy ou amino et facultativement substitué par ailleurs, tel que défini ci-dessus, dans lequel X représente un radical $-SOCH_2-$, l'oxydation d'un composé correspondant de formule (II) ou (III) tel que défini dans la revendication 2, dans lequel X représente un radical $-SCH_2-$;

e) pour la préparation d'un composé de formule (I) tel que défini dans la revendication 1, dans lequel $R^3$ est tel que défini ci-dessus, avec la restriction que le radical comprenant une base organique, un acide organique ou un sel d'ammonium quaternaire soit fixé au phényle via un oxygène ou un soufre, lequel procédé comprend la réaction d'un composé correspondant à un composé de formule (I) tel que défini dans la revendication 1, mais dans lequel $R^3$ représente un radical méta-hydroxyphényle ou méta-mercaptophényle, facultativement substitué par ailleurs tel que défini ci-dessus ou un racémique de celui-ci, avec un composé de formule $R^6Z$, dans lequel Z représente un atome ou un groupe partant, et $R^6$ représente un radical contenant une base organique, un acide organique ou un sel d'ammonium quaternaire, $R^6$ étant éventuellement sous forme protégée; après quoi :

i) tout groupe protecteur présent est éliminé pour former un composé de l'invention; et/ou

ii) quand un composé de formule (I) tel que défini dans la revendication 1 ou un racémique de celui-ci est obtenu sous la forme d'une base ou d'un acide, et qu'un sel est requis, la réaction de ce composé de formule (I) tel que défini dans la revendication 1, ou d'un racémique de celui-ci avec, respectivement, un acide ou une base pour former un sel.

f) pour la préparation d'un composé de formule (I) tel que défini dans la revendication 1, dans lequel $R^3$ est tel que défini ci-dessus, avec la restriction que le substituant en position méta soit un sel d'ammonium quaternaire et soit fixé au phényle via un oxygène, un -NH-ou un soufre, lequel procédé comprend la quaternisation d'un composé qui correspond à un composé de formule (I) tel que défini dans la revendication 1, mais dans lequel $R^3$ représente un radical contenant une amine tertiaire.

g) pour la préparation d'un composé de formule (I) tel que défini dans la revendication 1, dans lequel $R^3$ est tel que défini ci-dessus, avec la restriction que le substituant en position méta soit fixé au phényle via un -NH-, lequel procédé comprend la réaction d'un composé correspondant à un composé de formule (I) tel que défini dans la revendication 1, mais dans lequel $R^3$ représente un radical méta-aminophényle (facultativement substitué par ailleurs, tel que défini ci-dessus) ou d'un racémique de celui-ci, avec un composé de formule $R^6Z$ (où $R^6$ est tel que défini ci-dessus et Z représente un atome ou groupe partant ou un radical cyano), $R^6$ étant éventuellement sous forme protégée;

après quoi:

i) tout groupe protecteur présent est éliminé pour former un composé de l'invention; et/ou

ii) quand un composé de formule (I) tel que défini dans la revendication 1 ou un racémique de celui-ci est obtenu sous la forme d'une base ou d'un acide, et qu'un sel est requis, la réaction de ce composé de formule (I) tel que défini dans la revendication 1, ou d'un racémique de celui-ci avec, respectivement, un acide ou une base pour former un sel.

**14.** Composition pharmaceutique comprenant comme ingrédient actif au moins un composé de formule (I) tel que défini dans la revendication 1 ou un racémique de celui-ci ou un sel pharmaceutiquement acceptable de ce composé ou racémique, en association avec un excipient ou diluant pharmaceutique-

ment acceptable.

**Revendications pour l'Etat contractant suivant : ES**

**1.** Procédé pour préparer un composé de formule (I) :

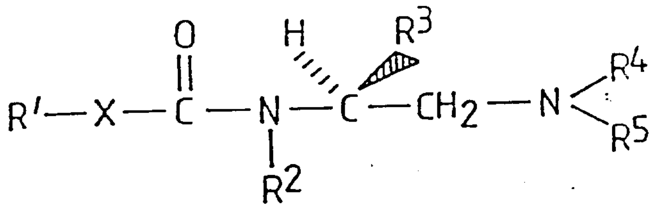

(I)

[où $R^1$ représente un radical halophényle, dihalophényle, nitrophényle, cyanophényle ou trifluorométhylphényle;

X représente une simple liaison, $-CH_2-$, $-OCH_2-$, $-SCH_2-$, $-S(O)-CH_2-$, $-S(O)_2-CH_2-$ ou $-CH_2-CH_2-$;

$R^2$ représente un hydrogène ou alcoyle en $C_{1-3}$;

$R^3$ représente un radical phényle substitué en position méta par un radical contenant une base organique, un acide organique ou un sel d'ammonium quaternaire et, facultativement, substitué par ailleurs par halogène, $C^{1-3}$ alcoyle, $C_{1-3}$ alcoxy, cyano, nitro, hydroxy, amino, mono- ou di-($C_{1\ ou\ 2}$ alcoyl)amino, carboxamido, mono- ou di-($C_{1\ ou\ 2}$ alcoyl)carboxamido, uréido ou $C_{1-3}$ acylamino;

$R^4$ et $R^5$, qui peuvent être les mêmes ou différents, représentent chacun un radical $C_{3-5}$ alcényle, $C_{3-5}$ alcynyle, $C_{1-6}$ alcoyle ou $C_{4-7}$ cycloalcoyle; ou

$R^4$ et $R^5$ représentent ensemble, avec l'atome d'azote intermédiaire, un hétérocycle à 4-7 membres, qui contient facultativement un hétéroatome supplémentaire choisi parmi oxygène et soufre]

ou le racémique de celui-ci, et les sels de ce composé ou racémique; le groupe $R^3$ étant choisi de manière à ce que le composé de formule (I) ou son racémique ou le sel de ce composé de formule (I) ou de son racémique ait un log D inférieur à 0,5 à pH 7,4 (où log D est le logarithme du coefficient de distribution entre le 1-octanol et un tampon aqueux); qui comprend :

a) pour la préparation d'un composé de formule (I) tel que défini ici, la réaction d'un composé de formule (IX)

$R^1$-X-$Y^2$ (IX)

dans lequel $R^1$ et X sont tels que définis ci-dessus et $Y^2$ représente un acide ou un dérivé activé de celui-ci, avec un composé de formule (X)

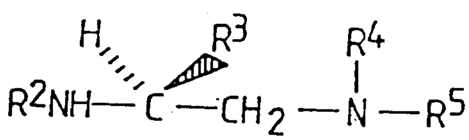

(X)

(où $R^2$, $R^3$, $R^4$ et $R^5$ sont tels que définis ci-dessus), ou d'un racémique de celui-ci, facultativement sous une forme protégée, et, quand c'est nécessaire, la déprotection du composé ainsi obtenu pour former un composé de formule (I) tel que défini ici ou un racémique de celui-ci, et, si désiré, la réaction de ce composé ou racémique avec un acide pour former un sel;

b) pour la préparation d'un composé de formule (I) tel que défini ici, dans lequel $R^3$ représente un radical phényle substitué en méta par hydroxy ou amino et facultativement substitué par ailleurs, comme défini ci-dessus, l'hydrolyse d'un composé correspondant, dans lequel ce radical phényle est substitué en méta par un radical acyloxy ou acylamino, ou par la déprotection d'un composé correspondant dans lequel ce radical phényle est substitué en méta par un radical alcoyloxy ou benzyle;

c) pour la préparation d'un composé de formule (I) tel que défini ici, dans lequel $R^3$ représente un radical phényle substitué en méta par hydroxy ou amino et facultativement substitué par ailleurs comme défini ci-dessus, dans lequel X représente un radical $-SOCH_2-$, l'oxydation d'un composé correspondant de formule (II) :

-Y-W-M (II)

ou (III):

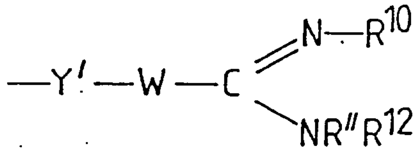

(III)

où X représente un radical $-SCH_2-$ ou $-SOCH_2-$;

d) pour la préparation de composés de formule (I) tel que défini ici, dans lequel $R^3$ représente un radical phényle substitué en méta par hydroxy ou amino et facultativement substitué par ailleurs, tel que défini ci-dessus, dans lequel X représente un radical $-SOCH_2-$, l'oxydation d'un composé correspondant de formule (II) ou (III) tel que défini ici où X représente un radical $-SCH_2-$;

e) pour la préparation d'un composé de formule (I) tel que défini ici, dans lequel $R^3$ est tel que défini ci-dessus, avec la restriction que le radical comprenant une base organique, un acide organique ou un sel d'ammonium quaternaire soit fixé au phényle via un oxygène ou un soufre, lequel procédé comprend la réaction d'un composé correspondant à un composé de formule (I) tel que défini ici, mais dans lequel $R^3$ représente un radical méta-hydroxyphényle ou méta-mercaptophényle, facultativement substitué par ailleurs tel que défini ci-dessus ou un racémique de celui-ci, avec un composé de formule $R^6Z$, dans lequel Z représente un atome ou un groupe partant, et $R^6$ représente un radical contenant une base organique, un acide organique ou un sel d'ammonium quaternaire, $R^6$ étant éventuellement sous forme protégée;

après quoi :

i) tout groupe protecteur présent est éliminé pour former un composé de l'invention; et/ou

ii) quand un composé de formule (I) tel que défini ici ou un racémique de celui-ci est obtenu sous la forme d'une base ou d'un acide, et qu'un sel est requis, la réaction de ce composé de formule (I) tel que défini ici, ou d'un racémique de celui-ci avec, respectivement, un acide ou une base pour former un sel.

f) pour la préparation d'un composé de formule (I) tel que défini ici, dans lequel $R^3$ est tel que défini ci-dessus, avec la restriction que le substituant en position méta soit un sel d'ammonium quaternaire et soit fixé au phényle via un oxygène, un -NH- ou un soufre, lequel procédé comprend la quaternisation d'un composé qui correspond à un composé de formule (I) tel que défini ici, mais dans lequel $R^3$ représente un radical contenant une amine tertiaire.

g) pour la préparation d'un composé de formule (I) tel que défini ici, dans lequel $R^3$ est tel que défini ci-dessus, avec la restriction que le substituant en position méta soit fixé au phényle via un -NH-, lequel procédé comprend la réaction d'un composé correspondant à un composé de formule (I) tel que défini ici, mais dans lequel $R^3$ représente un radical méta-aminophényle (facultativement substitué par ailleurs, tel que défini ci-dessus) ou d'un racémique de celui-ci, avec un composé de formule $R^6Z$ (où $R^6$ est tel que défini ci-dessus et Z représente un atome ou groupe partant ou un radical cyano), $R^6$ étant éventuellement sous forme protégée;

après quoi:

i) tout groupe protecteur présent est éliminé pour former un composé de l'invention; et/ou

ii) quand un composé de formule (I) tel que défini ici ou un racémique de celui-ci est obtenu sous la forme d'une base ou d'un acide, et qu'un sel est requis, la réaction de ce composé de formule (I) tel que défini la ici, ou d'un racémique de celui-ci avec, respectivement, un acide ou une base pour former un sel.

**2.** Procédé suivant la revendication 1, dans lequel $R^3$ représente phényle substitué en position méta par :

(i) un groupe de formule tel que défini ici :

-Y-W-M (II)

dans laquelle Y représente une liaison simple, -S-, -O- ou -NR- (où R représente hydrogène, méthyle, formyle ou acétyle), W représente une chaîne alcoylène en $C_{2-4}$, qui peut être branchée si désiré, et M représente le groupe $-NR^7R^8R^9$ (où $R^7$, $R^8$ et $R^9$, qui peuvent être les mêmes ou différents, représentent chacun un groupe alcoyle ou aralcoyle en $C_{1-7}$);

(ii) un groupe de formule (III), tel que défini dans la revendication 1, dans lequel $Y^1$ représente -O- ou -S-; W représente une chaîne alcoylène en $C_{2-4}$, qui peut être branchée si désiré, et $R^{10}$, $R^{11}$ et $R^{12}$, qui peuvent être les mêmes ou différents, représentent chacun hydrogène ou méthyle ou $R^{10}$ et $R^{11}$ représentent ensemble une chaîne alcoylène en $C_2$ ou en $C_3$, de manière que $R^{10}$ et $R^{11}$ forment, avec le radical intermédiaire de formule (IV) :

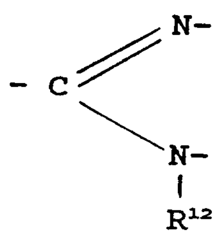

(IV)

un cycle 4,5-dihydroimidazol-2-yle ou 3,4,5,6-tétrahydro-pyrimidin-2-yle, $R^{12}$ représentant hydrogène ou méthyle; ou $R^{11}$ et $R^{12}$ représentent ensemble une chaîne alcoylène en $C_4$ ou en $C_5$, de manière que $R^{11}$ et $R^{12}$ forment avec l'atome d'azote intermédiaire un cycle pyrrolidine ou pipéridine et $R^{10}$ représente hydrogène ou méthyle;

(iii) un groupe de formule (V) :

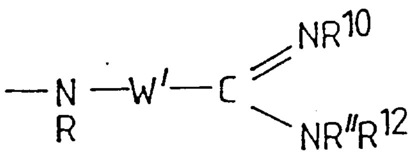

(V)

dans lequel $W^1$ représente une chaîne alcoylène en $C_{2-4}$, qui peut être branchée si désiré, et $R^{10}$, $R^{11}$ et $R^{12}$ sont tels que définis ci-dessus;

(iv) un groupe de formule (VI) :

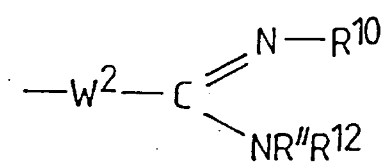

(VI)

dans lequel $W^2$ représente une chaîne alcoylène en $C_{1-4}$, qui peut être branchée si désiré, et $R^{10}$, $R^{11}$ et $R^{12}$ sont tels que définis ci-dessus;

(v) un groupe de formule (VII) :

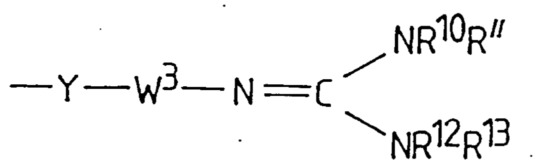

(VII)

dans lequel Y est tel que défini ci-dessus, $W^3$ représente une chaîne alcoylène en $C_{2-4}$, qui peut être branchée si désiré, ou, quand Y représente une liaison simple, $W^3$ peut représenter une liaison simple ou une chaîne alcoylène en $C_{1-4}$, qui peut être branchée si désiré, et $R^{10}$, $R^{11}$, $R^{12}$ et $R^{13}$, qui peuvent être les mêmes ou différents, représentent chacun hydrogène ou méthyle,

ou $R^{10}$ et $R^{12}$ représentent ensemble une chaîne alcoylène en $C_2$ ou en $C_3$, de manière que $R^{10}$ et $R^{12}$ forment, avec le radical intermédiaire de formule (IVa) :

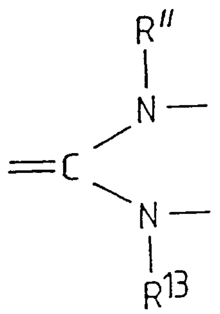

(IVa)

un cycle 4,5-dihydroimidazol-2-yle ou 3,4,5,6-tétrahydro-pyrimidin-2-yle, ou un tautomère correspondant, $R^{11}$ et $R^{12}$ étant choisis indépendamment parmi hydrogène et méthyle,

ou $R^{10}$ et $R^{11}$ ensemble, ou $R^{12}$ et $R^{13}$ ensemble représentent une chaîne alcoylène en $C_4$ ou en $C_5$, de manière que soit $R^{10}$ et $R^{11}$ forment avec l'atome d'azote intermédiaire un cycle pyrrolidine ou pipéridine et, $R^{12}$ et $R^{13}$ sont choisis indépendamment parmi hydrogène et méthyle, soit $R^{12}$ et $R^{13}$ forment avec l'atome d'azote intermédiaire un cycle pyrrolidine ou pipéridine et, $R^{10}$ et $R^{11}$ sont choisis indépendamment parmi hydrogène et méthyle; ou

(vi) un groupe de formule (VIII), dans lequel Y et $W^2$ sont tels que définis précédemment, et A représente un acide carboxylique ou sulfonique.

**3.** Procédé suivant la revendication 1 ou 2, dans lequel la base organique, l'acide organique ou le sel d'ammonium quaternaire est lié au phényle en position méta via un oxygène ou un -NH-.

**4.** Procédé suivant la revendication 1, 2 pu 3, dans lequel $R^1$ représente chlorophényle, dichlorophényle, bromophényle, fluorophényle, difluorophényle, trifluorométhylphényle, nitrophényle ou cyanophényle.

**5.** Procédé suivant l'une quelconque des revendications précédentes, dans lequel X représente $-CH_2-$, $-OCH_2-$, $-SCH_2-$, ou $-CH_2CH_2-$.

**6.** Procédé suivant l'une quelconque des revendications précédentes, dans lequel $R^3$ représente phényle substitué en position méta par :

i) un groupe de formule (II) tel que défini dans la revendication 1, dans lequel W est alcoylène en $C_2$ ou en $C_3$;

ii) un groupe de formule (III) tel que défini dans la revendication 1, dans lequel W est $-CH_2CH_2-$;

iii) un groupe de formule (V) tel que défini dans la revendication 1, dans lequel $W^1$ est une liaison simple ou $-CH_2CH_2-$;

iv) un groupe de formule (VI) tel que défini dans la revendication 1, dans lequel $W^2$ est $-CH_2-$ ou $-CH_2CH_2-$;

v) un groupe de formule (VII) tel que défini dans la revendication 1, dans lequel Y représente -O-, -S-ou -NR-
(où R est tel que défini ci-dessus) et $W^3$ est $-CH_2CH_2-$; ou un groupe de formule (VII) dans lequel Y représente une liaison simple et $W^3$ est une liaison simple, $-CH_2-$ ou $-CH_2CH_2-$; ou
vi) un groupe de formule (VIII) tel que défini dans la revendication 2, dans lequel $W^2$ est $-CH_2-$.

**7.** Procédé suivant l'une quelconque des revendications précédentes, dans lequel $R^3$ représente 3-(3-diméthylaminopropoxy)phényle, sous la forme d'un sel d'ammonium quaternaire, 3-(3-carboxyméthoxy)phényle, 3-guanidinophényle; ou un radical 3-triméthylammoniumpropoxy ou 3-benzyldiméthylammoniumpropoxy stabilisé par un anion approprié.

**8.** Procédé suivant l'une quelconque des revendications précédentes, dans lequel $R^4$ et $R^5$, ensemble avec l'atome d'azote intermédiaire, représentent un radical pyrrolidino, diméthylamino, diéthylamino, N-allyl-M-méthylamino, N-isopropyl-N-méthylamino, $\Delta^3$-pyrrolino ou pipéridino.

**9.** Procédé suivant la revendication 1, qui comprend la préparation de la 2(R,S)-N-[2-(N-méthyl-3,4-dichlorophénylacétamido)-2-(3-carboxyméthoxyphényl)-éthyl]pyrrolidine, de l'iodure de (R,S)-N-[2-(N-méthyl-3,4-dichlorophénylacétamido)-2-(3-triméthylammoniumpropoxyphényl)-éthyl]pyrrolidine,du chlorure de (R,S)-N-[2-(N-méthyl-3,4-dichlorophénylacétamido)-2-(3-benzyldiméthylammoniumpropoxyphényl)-éthyl]pyrrolidine; ou de la (R,S)-N-[2-(N-méthyl-3,4-dichlorophénylacétamido)-2-(3-guanidinophényl)-éthyl]pyrrolidine et des sels de ceux-ci.

**10.** Procédé suivant l'une quelconque des revendications précédentes, dans lequel le composé de formule (I) tel que défini dans la revendication 1 est sous la forme d'un sel pharmaceutiquement acceptable.